# EUROPEAN PATENT APPLICATION

(11) **EP 3 862 014 A1**
(43) Date of publication of application: **11.08.2021**
(21) Application number: 20156088.5
(22) Date of filing: 07.02.2020
(51) Int. Cl.: A61K 38/08, A61K 38/16

(54) **TREATMENT OF PANX1 ASSOCIATED DISEASES**

(71) Applicant: Nuritas Limited, D02 RY95 Dublin 2 (IE)
(72) Inventor: Khaldi, Nora, Co. Dublin, (IE); Lopez, Cyril, Dublin 2 (IE)
(74) Representative: Purdylucey Intellectual Property

(57) **Abstract**

The inventors have discovered that the peptide of SEQUENCE ID NO: 1 (WKDEAGKPLVK) targets PANX1. The invention also concerns treatment of diseases associated with PANX1 with the peptide or a composition comprising the peptide.

## Description

### Field of the Invention

The current invention relates to treatment or prevention of PANX1 associated diseases, in a subject.

### Background to the Invention

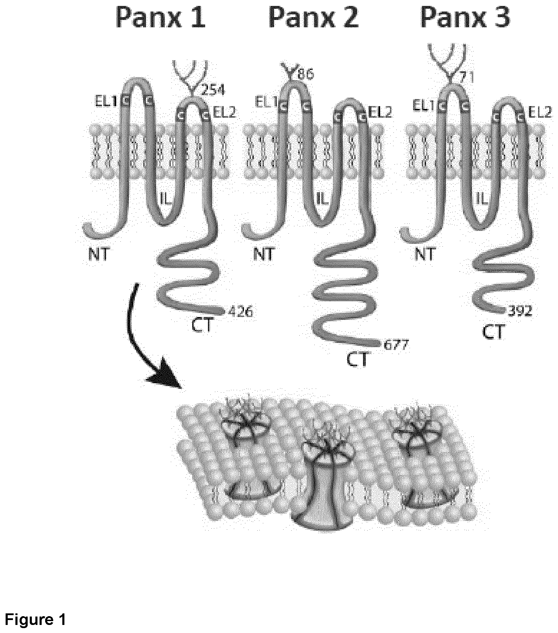

Pannexin 1 (PANX1) is a protein encoded by the PANX1 gene and a member of the Pannexin family. The pannexin family is a family of transmembrane (TM) channel proteins with three members, namely PANX1, PANX2 and PANX3. Each family member comprises four α-helical TM domains, two extracellular loops and one intracellular loop (Figure 1). The N and C termini of the protein are exposed to the cell's cytoplasm. The protein provides a membrane channel pore of 9Å that allows Ca2+, K+, ATP and molecules ∼1.2kDa to enter and exit the cell. PANX2 and PANX3 expression is largely restricted to the central nervous system (CNS), fibroblasts and osteoblasts. PANX1 is expressed in most cell types, including the liver, kidney, lung GI tract and the pancreas. PANX1 is localised primarily at the plasma membrane of cells.

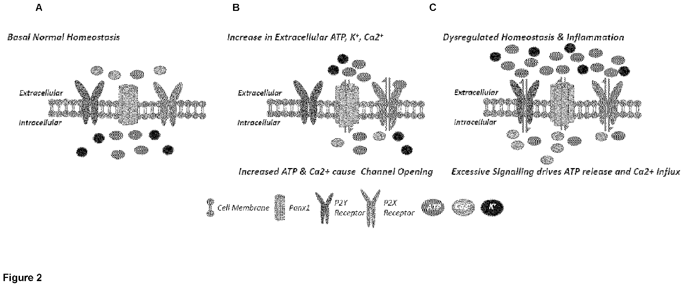

PANX1 channels release adenosine triphosphate (ATP) and play a role in many normal pathological processes in a wide range of cell and tissue types. ATP is an extracellular signalling molecule providing energy to drive many cell processes. Disease states, e.g. cell damage, hyperlipidaemia, hypoxia, chronic inflammation and/or infection, drive extracellular (EC) ATP production. An increase in inflammatory signals EC ATP production causes an increases PANX1 channel opening. This stimulates ATP cell efflux and Ca2+ cell influx (Figure 2). This excessive PANX1 signalling stimulates inflammasome assembly, facilitating the release of pro-inflammatory cytokines, such as interleukin (IL)-1β and IL-18. This in turn leads to caspase activation. Dysregulated pro-inflammatory cytokines can also in turn activate factors, such as TFG β, TNFα, PDGF.

PANX1 has been implicated in multiple diseases and conditions, including ischemia, pain, fibrosis, microbial infection, inflammation and cancer, and many groups have studied PANX1 as a target in disease treatment. Studies have shown that PANX1 is related to epilepsy, neuropathic pain, painful musculoskeletal diseases, ischemia injury, myocardial fibrosis, HIV infection, overactivity of the human bladder, and cancers (Di Wu, et al., Acta Biochim Biophys Sin, 2016). Elevated or hyperactive PANX1 levels have also been associated disease that include melanoma, ischemic stroke, seizures, colitis migraine, headaches, osteoarthritis epilepsy, among others (Laird et al., Nat Rev Drug Discov., 2018).

Good, et al., (Circulation Research, 2017) disclosed therapeutic modalities for resistant hypertension based on PANX1 inhibition. This group researched PANX1 as an *in vivo* target of the hypertension drug spironolactone. Other groups have implicated mutated forms of PANX1 in highly metastatic cancer cells. Mutated PANX1 augmented ATP release and enhanced efficiency of metastasis by promoting breast cancer cell survival in a study (Furlow PW, Nat Cell Biol, 2015). In this study, a PANX1 inhibitor was shown to reduced metastasis.

PANX1-targeted therapy has been investigated as an approach for alleviating joint pain (Mousseu et al., Neurophysiology Science Advances 8 Aug 2018). Probenecid, a drug for the treatment of gout, has been shown to attenuate PANX1 channel-induced ATP (Silverman W., Am J Physiol Cell Physiol, 2008). Carbenoxolone (CBX), a PANX1 channel inhibitor, is a drug for gastric ulcers (Benefenati V, Channels., 2009) and has entered trials for Huntington's disease.

PANX1 channels have been investigated as a therapeutic target in opiate withdrawal. This group found that blocking PANX1 alleviates the severity of withdrawal without affecting opiate analgesia (Burma et al., Nature Medicine, 2017). Makarenkova HP, et al., suggest that pannexin reduction may be an effective strategy to reduce pain and promote regeneration after nerve injury and implicate PANX1 signalling pathways in suppression of inflammation.

Feig et al., (PLoS ONE 12, 2017) reported that the antiviral drug, tenofovir, an inhibitor of PANX1 mediated ATP release, prevents liver and skin fibrosis by downregulating adenosine levels in the liver and skin. Crespo Yanguas et al, (Arch Toxicol, 2018) investigated the role of PANX1 in liver fibrosis and reported chemical induced liver fibrosis increased PANX1 expression and that PANX1 ablation attenuates CCL4 induced liver fibrosis.

Oritiz et al., (Perspective 2020) reported the role of pannexin 1 based channels as a regulator of multiple sclerosis progression.

The current invention provides an agent that surprisingly targets PANX1 for the treatment or prevention of PANX1 associated disease.

### Summary of the Invention

The inventors have surprisingly discovered that the peptide of SEQUENCE ID NO. 1. (WKDEAGKPLVK) targets PANX1. The peptide of the invention provides a means to modulate PANX1 or PANX1 signalling and can be used to treat or prevent diseases or conditions which are associated with PANX1. In an embodiment, the peptide of the invention elicits its effect by blocking or inhibiting PANX1 or PANX1 signalling.

Accordingly, in a first aspect, the invention provides a peptide comprising SEQUENCE ID NO. 1, or a functional (or therapeutically effective) variant of SEQUENCE ID NO. 1 (hereafter "peptide active agent" or "peptide of the invention"), for use in a method for the treatment or prevention of a disease or condition associated with PANX1, in a subject.

In a further aspect, the invention provides a composition comprising a peptide comprising SEQUENCE ID NO: 1, or a therapeutically effective variant of SEQUENCE ID NO: 1, for use in a method for the treatment or prevention of a disease or condition associated with PANX1, in a subject.

Suitably, the composition comprises a plurality of peptides.

In another aspect, the invention relates to a method for the treatment or prevention a disease or condition associated with PANX1, in a subject comprising a step of administering to the subject a therapeutically effective amount of a peptide comprising SEQUENCE ID NO: 1, or a functional (or therapeutically effective) variant of SEQUENCE ID NO: 1 (hereafter "peptide active agent").

The functional (or therapeutically effective) variant may be a functional or therapeutic fragment of SEQUENCE ID NO. 1.

Preferably, the disease or condition may be one selected from the group comprising an inflammatory disease or condition, fibrotic disease, cancer, ischemia and cardiovascular disease.

Preferably, the disease or condition may be one selected from the group comprising fibrosis, melanoma, liver cancer, ischemia, hypertension, and multiple sclerosis.

The disease may include, but is not limited to, inflammatory bowel disease, such as Crohn's disease, chronic gastrointestinal (GI) inflammation, sepsis, hepatic fibrosis, skin fibrosis, renal fibrosis, pulmonary fibrosis, melanoma, breast carcinoma, hepatocellular carcinoma, brain ischemia, ischemic stroke, pain, cardiomyocyte fibrosis, microbial infection, brain inflammation, hypertension, a neurodegenerative disease and a peripheral nerve disease.

Preferably, the peptide is up to 50 amino acids in length. In one embodiment, the peptide has up to 40, 35, 30, 25, 20, or 15 amino acids. In one embodiment, the peptide has from 11 to 15 amino acids.

The peptide (or functional variant or fragment) may be administered alone or in combination with other co-drugs that provide an enhanced therapeutic effect, which include but not limited to, drugs which have identified effect in the treatment of the disease or condition associated with PANX1.

In one embodiment, the peptide consists essentially of SEQUENCE ID NO: 1.

In one embodiment, the variant of the peptide has from 1 to 8 modifications or alterations compared with SEQUENCE ID NO: 1, the modification typically independently selected from insertion, addition, deletion, and substitution (ideally conservative substitution) of an amino acid.

In one embodiment, one or more amino acids (for example 1 to 5, 1 to 4, 1 to 3, or 1 to 2) are replaced with D-amino acids. In one embodiment, one or more of residues 1, 2, 5, 10, 11 are replaced with D-amino acids, for example 2, 3, 4, or 5 of the residues. In one embodiment, one or more amino acids are replaced with conservative amino acid substitutions.

In one embodiment, the functional or therapeutic variant has a sequence {d}W{d}KDE{d}AGKPL{d}V{d}K (SEQUENCE ID NO. 86), which is the same as SEQUENCE ID NO. 1 with the exception that amino acids 1, 2, 5, 10 and 11 are replaced with D-form of the amino acid.

In one embodiment, the peptide is modified. In one embodiment, the peptide is a recombinant peptide. In one embodiment, the peptide is cyclised.

Preferably, the peptide is modified by a modification(s) to side chains, incorporation of unnatural amino acids and/or their derivatives during peptide synthesis, the use of cross-linkers and other methods that impose conformational constraint on the peptide, by conjugation to a conjugation partner, by fusion to a fusion partner, covalent linkage to a binding partner, lipidation, PEGylation, and amidation.

An example of a cyclised peptide is (1(clac)wKE(Me)EC1GK(Me)PLVk-OH) - SEQUENCE ID NO. 87. In this variant, the residues "w" and ""k" are D-amino acids, the residues "E" and "P" are methylated, and the peptide comprises a thioether cyclization between the n-terminus and the cysteine residue, in which "1(clac)" and "C1" indicate the two ends of the cycle.

Other aspects and preferred embodiments of the invention are defined and described in the other claims set out below.

### Brief Description of the Figures

The invention will now be described with reference to the accompanying Figures in which;
**Figure 1** illustrates PANX1, PANX2 and PANX3 transmembrane proteins.
**Figure 2** **A, B and C** illustrate activity of PANX1 in normal homeostasis (A), in the presence of an increase in extracellular ATP and K+ (B), and in the presence of dysregulated homeostasis and inflammation (C).
**Figure 3** illustrates binding affinity of the peptide of the invention (peptide of SEQUENCE ID NO. 1) to human membrane proteins. CY5-labelled peptide (0.01 & 0.05 µg/ml) was screened against HEK293 cells expressing 5528 human plasma membrane proteins.
**Figure 4** illustrates peptide of SEQUENCE ID NO. 1 reduces the secretion of IL-8 in immortal hepatocyte cells. HepG2 cells were treated with peptide (5 ng/ml) for 24 h before treating with 100 ng/mL of LPS for 24 h. Data presented are the mean ± SEM of at least 2 independent experiments. (*p<0.05 **p<0.01 ***p<0.001)
**Figure 5** **A and B** illustrate peptide of SEQUENCE ID NO. 1 shows anti-fibrotic activity in primary human hepatic stellate cells. (A) Confocal imaging of human stellate cells treated with TGF-β1 to stimulate expression of α-SMA before treatment with peptide (5 nM). (B) Quantification of α-SMA expression.
**Figure 6** illustrates the various cellular processes mediated by PANX1 signalling.
**Figure 7** **A and B** illustrate intracellular concentrations of peptide before (A) and after (B) channel activation. when the channel is activated.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

The peptide of the invention has surprisingly been found to target and bind to PANX1 protein in tissue and modulate cellular processes associated with PANX1 signalling. In this manner, the peptide of the invention can be used to treat diseases and conditions that are associated with PANX1.

The amino acid sequence of PANX1 (isoform 1) is as follows:

MAIAQLATEYVFSDFLLKEPTEPKFKGLRLELAVDKMVTCIAVGLPLLLISLAFAQEISIGTQIS
CFSPSSFSWRQAAFVDSYCWAAVQQKNSLQSESGNLPLWLHKFFPYILLLFAILLYLPPLFW
RFAAAPHICSDLKFIMEELDKVYNRAIKAAKSARDLDMRDGACSVPGVTENLGQSLWEVSE
SHFKYPIVEQYLKTKKNSNNLIIKYISCRLLTLIIILLACIYLGYYFSLSSLSDEFVCSIKSGILRND
STVPDQFQCKLIAVGIFQLLSVINLVVYVLLAPVVVYTLFVPFRQKTDVLKVYEILPTFDVLHF
KSEGYNDLSLYNLFLEENISEVKSYKCLKVLENIKSSGQGIDPMLLLTNLGMIKMDVVDGKTP
MSAEMREEQGNQTAELQGMNIDSETKANNGEKNARQRLLDSSC (SEQUENCE ID NO. 88)

Isoform 2 of PANX1 has the following sequence:

MAIAQLATEYVFSDFLLKEPTEPKFKGLRLELAVDKMVTCIAVGLPLLLISLAFAQEISIGTQIS
CFSPSSFSWRQAAFVDSYCWAAVQQKNSLQSESGNLPLWLHKFFPYILLLFAILLYLPPLFW
RFAAAPHICSDLKFIMEELDKVYNRAIKAAKSARDLDMRDGACSVPGVTENLGQSLWEVSE
SHFKYPIVEQYLKTKKNSNNLIIKYISCRLLTLIIILLACIYLGYYFSLSSLSDEFVCSIKSGILRND
STVPDQFQCKLIAVGIFQLLSVINLVVYVLLAPVVVYTLFVPFRQKTDVLKVYEILPTFDVLHF
KSEGYNDLSLYNLFLEENISEVKSYKCLKVLENIKSSGQGIDPMLLLTNLGMIKMDVVDGKTP
MSAEMREEQGNQTAELQDSETKANNGEKNARQRLLDSSC (SEQUENCE ID NO. 89)

The nucleotide sequence for PANX1 has the NCBI Reference Sequence Accession No. NG_027936.1

The disease or condition is one associated with, or mediated by, PANX1 involvement. The disease or condition may be one associated with, or characterised by, an increase in PANX1 signalling or expression in a subject, or biological sample of a subject.

Of note, the disease or condition may be selected from the group comprising inflammation, fibrosis, cancer, ischemia and cardiovascular disease. The disease or condition may be pain, a disease or condition associated with the nervous system. The disease or condition may be one associated with the brain.

The inflammation may be any type of inflammation or inflammatory disease or condition, such as one characterised by a chronic inflammatory environment. The inflammation may be cerebral inflammation, intestinal inflammation, brain inflammation, stomach inflammation or vascular inflammation. The inflammation may be that caused by an injury. The inflammation may include but is not limited to inflammatory bowel disease, such as Crohn's disease and chronic gastrointestinal (GI) inflammation.

The fibrotic disease or condition, or fibrosis, may be or involve any tissue. Suitable examples include but are not limited to hepatic fibrosis, renal fibrosis, pulmonary fibrosis, cardiomyocyte fibrosis, myocardial fibrosis, bone marrow fibrosis, kidney fibrosis, tissue fibrosis and skin fibrosis.

The cancer may include but is not limited to melanoma, breast cancer, liver cancer including hepatocellular cancer, lung cancer, testicular cancer, glioma, multiple myeloma, colon cancer, leukaemia and endometrial cancer.

The cardiovascular disease may include but is not limited to coronary heart disease, stroke, peripheral vascular disease, cardiomyopathy and atherosclerosis. In one embodiment, the cardiovascular disease does not include atherosclerosis.

The disease or condition may be resistant hypertension.

The ischemia may include but is not limited to brain ischemia, ischemic stroke, cardiac ischemia, bowel ischemia, limb ischemia, and ischemia caused by trauma or occlusion.

The disease or condition may be one of the nervous system, including peripheral nerve disease. The disease or condition may be a neurodegenerative disease, such as multiple sclerosis.

The disease or condition may sepsis or pain. The pain may be any part of the subject. The pain may be joint pain.

The condition may be an injury.

The disease or condition may be an ophthalmic disease or condition.

The disease or condition may be a microbial infection such as a bacterial infection, a viral infection or a fungus infection. The infection may be a HIV infection.

The condition may be withdrawal from a drug, such as an opioid.

The disease or condition may be a musculoskeletal disorder, such as tendonitis, or carpal tunnel syndrome.

The disease or condition may be selected from the group comprising migraine, headache, seizures, osteoarthritis epilepsy, lesional epilepsy, drug-resistant epilepsies and epilepsy.

The disease or condition may be a kidney disease or bladder disease, including overactivity of the bladder. The condition may be acute kidney injury.

The disease or condition may be a gastric ulcer. The disease or condition may be gout. The disease or condition may be Huntington's disease.

The invention provides a peptide comprising SEQUENCE ID NO. 1, or a functional (or therapeutically effective) variant of SEQUENCE ID NO: 1 (hereafter "peptide active agent" or "peptide of the invention"), for use in a method for the treatment or prevention of a disease or condition associated with PANX1, in a subject.

The invention provides a composition comprising a peptide comprising SEQUENCE ID NO: 1, or a therapeutically effective variant of SEQUENCE ID NO: 1, for use in a method for the treatment or prevention of a disease or condition associated with PANX1, in a subject.

Notably, the composition is a man-made composition.

The composition may be a pharmaceutical composition and further comprise at least one suitable pharmaceutical carrier.

The variant may be a functional or therapeutic fragment of SEQUENCE ID NO. 1.

In one embodiment, the variant has 1 to 8 amino acid changes or modifications compared to SEQUENCE ID NO: 1. In one embodiment, the variant has 1 to 7 amino acid changes compared to SEQUENCE ID NO: 1. In one embodiment, the variant has 1 to 6 amino acid changes compared to SEQUENCE ID NO: 1. In one embodiment, the variant has 1 to 5 amino acid changes compared to SEQUENCE ID NO: 1. In one embodiment, the variant has 1 to 4 amino acid changes compared to SEQUENCE ID NO: 1. In one embodiment, the variant has 1 to 3 amino acid changes compared to SEQUENCE ID NO: 1. In one embodiment, the variant has 1 to 2 amino acid changes compared to SEQUENCE ID NO: 1. In one embodiment, the amino acid change is an amino acid substitution. In one embodiment, the amino acid substitution is a conservative substitution. In one embodiment, the amino acid change is an amino acid addition. In one embodiment, the amino acid change is an amino acid deletion.

The variants of the invention include: -

### Variants of SEQUENCE ID NO: 1

Variants of SEQUENCE ID NO: 1 (WKDEAGKPLVK) including variants having 1,2 or 3 conservative amino acid substitutions, 1, 2 to 3 non-conservative amino acid substitutions, 1, 2 or 3 amino acid additions, 1, 2 or 3 amino acid deletions, are provided below:

### One conservative amino acid substitution:

WKEEAGKPLVK (SEQ ID NO.2); FKDEAGKPLVK (SEQ ID NO.3); WKDEAGKPMVK (SEQ ID NO.4); WKDEAGRPLVK (SEQ ID NO.5); WRDEAGKPLVK (SEQ ID NO.6); WKDEAGKPLMK (SEQ ID NO.7); WKDQAGKPLVK (SEQ ID NO.8); WKDEATKPLVK (SEQ ID NO.9)

### Two conservative amino acid substitutions:

YKNEAGKPLVK (SEQ ID NO.10); WKNESGKPLVK (SEQ ID NO.11); WKDEAGKTLVR (SEQ ID NO.12); FKDEATKPLVK (SEQ ID NO. 13); FKDEAGKPLIK (SEQ ID NO. 14); WKDEAGKTLLK (SEQ ID NO. 15); WKNEAGKPVVK (SEQ ID NO.16); WKDEAGRTLVK (SEQ ID NO.17)

### Three conservative amino acid substitutions:

WEDESGKPLLK (SEQ ID NO.18); WKEEAGKPIVQ (SEQ ID NO.19); YKNEAGKPLVR (SEQ ID NO.20); WKDQATRPLVK (SEQ ID NO. 21); WKDESGKPVLK (SEQ ID NO.22); WQDDSGKPLVK (SEQ ID NO.23); WKNEAGKTLLK (SEQ ID NO.24); WKDKAGEPLVR (SEQ ID NO.25)

### One non-conservative amino acid substitution:

WKDEAGNPLVK (SEQ ID NO. 26); CKDEAGKPLVK (SEQ ID NO.27); WKDEAGKPLGK (SEQ ID NO.28); WKDENGKPLVK (SEQ ID NO.29); WKDEARKPLVK (SEQ ID NO.30); WKDEAGKPLVT (SEQ ID NO.31); WKDEAGKRLVK (SEQ ID NO.32); WKWEAGKPLVK (SEQ ID NO.33)

### Two non-conservative amino acid substitution:

WKDEAGFPTVK (SEQ ID NO. 34); WYDMAGKPLVK (SEQ ID NO. 35); WKDYEGKPLVK (SEQ ID NO. 36); WKREAGKPGVK (SEQ ID NO. 37); WKLEKGKPLVK (SEQ ID NO. 38); WKDEAGKPCVK (SEQ ID NO. 39); WKKEAPKPLVK (SEQ ID NO. 40); SKDEAGPPLVK (SEQ ID NO. 41)

### Three non-conservative amino acid substitution:

WKHEPGKPLAK (SEQ ID NO. 42); WKDEREKPFVK (SEQ ID NO. 43); WKQEAGKPWRK (SEQ ID NO. 44); VKDEAKKPLVH (SEQ ID NO. 45); NWDEAGKMLVK (SEQ ID NO. 46); IKDEDGPPLVK (SEQ ID NO. 47); LKDEYGKPLVN (SEQ ID NO. 48); WKDRAGKELTK (SEQ ID NO. 49)

### Amino acid additions

WKDEAGKPLPVK (SEQ ID NO. 50); WKGDENYAGKPLVK (SEQ ID NO.51); LWKDEAGRKYPLVK (SEQ ID NO.52); WKDCEGAGKPLVK (SEQ ID NO.53); WKDEPAGKPLVVK (SEQ ID NO.54); WKDEAGPKPLVK (SEQ ID NO.55); WKDEAGWADKPLVK (SEQ ID NO.56); WKNDEAGKPLVK (SEQ ID NO.57)

### Amino acid deletions

WKDAKPLVK (SEQ ID NO. 58); WKEAGKPVK (SEQ ID NO. 59); WKDEAKPLVK (SEQ ID NO. 60); WDEAGKPV (SEQ ID NO. 61); WKDEAGKPVK (SEQ ID NO. 62); WDAGKPLVK (SEQ ID NO. 63); WKDEAGKPLV (SEQ ID NO. 64); WEAGKPLV (SEQ ID NO. 65)

### Examples of fragments of SEQ ID NO: 1 are provided below:

WKDEAG (SEQ ID NO. 66); WKDEA (SEQ ID NO. 67); KDEAGKPL (SEQ ID NO. 68); KDEAG (SEQ ID NO. 69); DEAGKPL (SEQ ID NO. 70); GKPLV (SEQ ID NO. 71); DEAGK (SEQ ID NO. 72); WKDEAGKPL (SEQ ID NO. 73); WKD (SEQ ID NO. 74); KDE (SEQ ID NO. 75); KPLVK (SEQ ID NO. 76); WKDE (SEQ ID NO. 77); AGKPL (SEQ ID NO. 78); EAG (SEQ ID NO. 79); AGK (SEQ ID NO. 80); KPL (SEQ ID NO. 81); LVK (SEQ 20 ID NO. 82); GKP (SEQ ID NO. 83); DEA (SEQ ID NO. 84); PLV (SEQ ID NO. 85)

It will be appreciated that the composition may comprise a plurality of peptides, fragments and/or variants. Preferably, the composition comprises at least two peptides of the invention. Preferably, the composition comprises at least three peptides of the invention. Preferably, the composition comprises at least four peptides of the invention. Preferably, the composition comprises at least five peptides of the invention. Preferably, the composition comprises at least six peptides of the invention. Preferably, the composition comprises at least seven peptides of the invention. Preferably, the composition comprises at least eight peptides of the invention. Preferably, the composition comprises at least nine peptides of the invention. Preferably, the composition comprises at least ten peptides of the invention. In one embodiment, the composition comprises substantially all the peptides. In one embodiment, the composition comprises substantially all the variants. In one embodiment, the composition is substantially free of other peptides.

Figure 1 illustrates PANX1 channel protein. The protein comprises four α-helical TM domains, two extracellular loops and one intracellular loop. The N and C termini of the protein are exposed to the cell's cytoplasm.

Figure 2 illustrates the activity of PANX1 in normal homeostasis (A). This Figure also illustrates the activity of PANX1 in the presence of an increase in extracellular ATP and K+ (B). In this environment there is an increase in channel opening. Inflammation and dysregulated homeostasis drive excessive signalling with increases ATP release and Ca2+ cell influx.

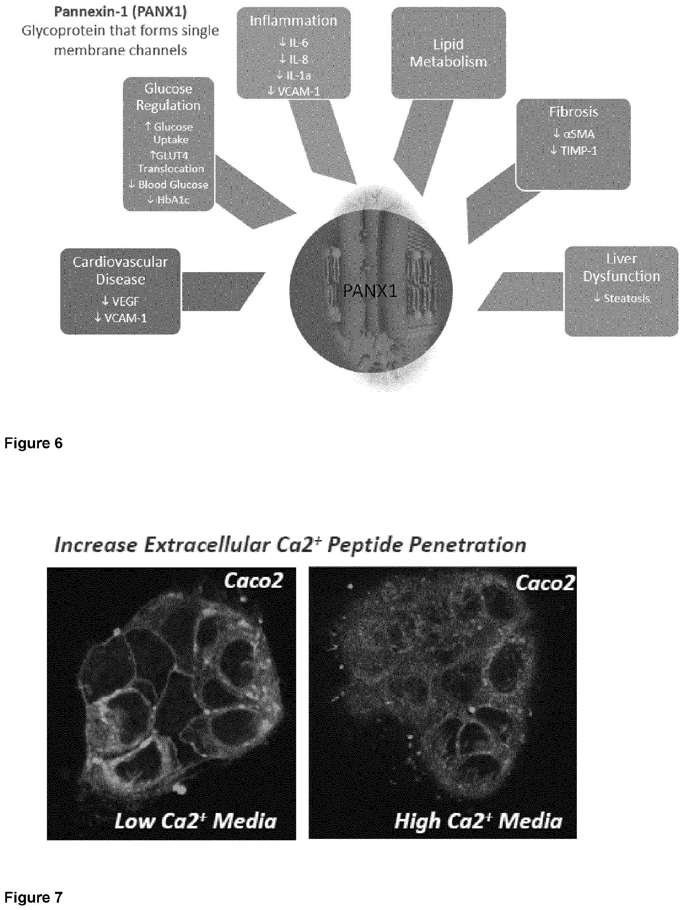

Figure 6 provides examples of the cellular process and/or diseases mediated by PANX1 or with PANX1 involvement. It will be appreciated that all included are envisaged herein in the current invention.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa*. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the phrase "a disease associated with PANX1" refers to a disease or condition in which PANX1 or PANX1 signalling plays a role. The disease or condition may be one which is characterised by or mediated by an increase in PANX1 signalling or PANX1 expression in a subject, or a biological sample of a subject. The increase is when compared with a healthy subject. The sample may be any biological sample, such as blood, tissue, cell, organ. In an embodiment, PANX1 is mutated in said subject such that PANX1 signalling and/or expression is increased compared with PANX1 that is not mutated. Such diseases and conditions are known in the art and all are envisaged herein. Means to detect PANX1 expression and signalling are well known to a person skilled in the art.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, "an effective amount " or "a therapeutically effective amount" of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the subject's physical size, age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term "subject" (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae*, which includes horses, donkeys, asses, kiang and zebra. The subject may be a subject in need thereof, i.e. in need to treatment or prevention of a disease or condition.

"Pharmaceutical compositions": A further aspect of the invention relates to a pharmaceutical composition comprising a peptide active agent, admixed with one or more pharmaceutically acceptable diluents, excipients or carriers, or co-administered with other drugs which enhance the therapeutic effect. Even though the peptide and composition of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p hydroxybenzoic acid. Antioxidants and suspending agents may be also used.

The term "peptide" used herein refers to a polymer composed of up to 50 amino acids, for example 5 to 50 amino acid monomers typically linked via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH4; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH4. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids. Changes may be those that reduce susceptibility to proteolysis, reduce susceptibility to oxidation, alter binding affinity of the variant sequence (typically desirably increasing affinity), and/or confer or modify other physicochemical or functional properties on the associated variant/analog peptide

The term "therapeutically effective variant" as applied to a reference peptide means peptides having an amino acid sequence that is substantially identical to the reference peptide, and which is therapeutically effective as defined below. Thus, for example, the term should be taken to include variants that are altered in respect of one or more amino acid residues. Preferably such alterations involve the insertion, addition, deletion and/or substitution of 6 or fewer amino acids, preferably 5 or fewer, 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 50%, 60%, 70% amino acid sequence identity, preferably at least 80% sequence identity, more preferably at least 90% sequence identity, and ideally at least 95%, 96%, 97%, 98% or 99% sequence identity with the parent sequence. It should be noted that any variant will have principally the same therapeutic effect, or may have enhanced effect, when tested in *in vitro* or *in* vivo models of the disease. An exemplary variant in which five of the amino acids are replaced with D-forms of the amino acids is provided as SEQUENCE ID NO: 86.

"Therapeutically effective", used interchangeably with "functional", as applied to a peptide of the invention means a peptide that is capable of targeting PANX1 and modulating its function. Means to determine targeting of PANX1 are as described herein. Means to determine PANX1 modulation are as described herein and as known in the field.

The term variant is also taken to encompass the term "fragment" and as such means a segment of amino acid SEQUENCE ID NO. 1. Typically, the fragment has between 3 and 10 contiguous amino acids in length. Generally, the fragment has a charge of -5 to +3. The charge of a peptide, fragment or region is determined using the method of Cameselle, J.C., Ribeiro, J.M., and Sillero, A. (1986). Derivation and use of a formula to calculate the net charge of acid-base compounds. Its application to amino acids, proteins and nucleotides. Biochem. Educ. 14, 131-136.

In this specification, the term "sequence identity" should be understand to comprise both sequence identity and similarity, i.e. a variant (or homolog) that shares 70% sequence identity with a reference sequence is one in which any 70% of aligned residues of the variant (or homolog) are identical to, or conservative substitutions of, the corresponding residues in the reference sequence across the entire length of the sequence. Sequence identity is the amount of characters which match exactly between two different sequences. Hereby, gaps are not counted and the measurement is relational to the shorter of the two sequences.

In terms of "sequence homology", the term should be understood to mean that a variant (or homolog) which shares a defined percent similarity or identity with a reference sequence when the percentage of aligned residues of the variant (or homolog) are either identical to, or conservative substitutions of, the corresponding residues in the reference sequence and where the variant (or homolog) shares the same function as the reference sequence.

This alignment and the percent homology or sequence identity can be determined using software programs known in the art, for example, one alignment program is BLAST, using default parameters. Details of these programs can be found at the following Internet address: http://www.ncbi.nlm.nih.gov/blast/Blast.cgi.

"C-terminal domain" as applied to a fragment means the first three amino acids at the c-terminus of the fragment.

"N-terminal domain" as applied to a fragment means the last three amino acids at the n-terminus of the fragment.

"Homolog" of a reference protein should be understood to mean a protein from a different species of plant having at least 60%, 70%, 80%, 90%, 91%, 92%, 93%, 94%, 95%, 96%, 97%, 98%, 99% sequence homology with the reference protein.

The peptide or composition may be adapted for, and administered by, topical, oral, rectal, parenteral, intramuscular, intraperitoneal, intra-arterial, intrabronchial, subcutaneous, intradermal, intravenous, nasal, vaginal, buccal or sublingual routes of administration. For oral administration, particular use is made of compressed tablets, pills, tablets, gellules, drops, and capsules. Preferably, these compositions contain from 1 to 250 mg and more preferably from 10-100 mg, of active ingredient per dose. Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders. The composition of the invention may be formulated for topical delivery. Topical delivery generally means delivery to the skin but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required. Injectable forms may contain between 10-1000 mg, preferably between 10-250 mg, of active ingredient per dose. Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention. Depending upon the need, the agent may be administered at a dose of from 0.01 to 30 mg/kg body weight, such as from 0.1 to 10 mg/kg, more preferably from 0.1 to 1 mg/kg body weight. In an exemplary embodiment, one or more doses of 10 to 300 mg/day or more preferably, 10 to 150 mg/day, will be administered to the patient for the treatment of an inflammatory disorder.

In a particularly preferred embodiment, the methods and uses of the invention involve administration of a peptide or composition in combination with one or more other active agents, for example, existing drugs or pharmacological enhancers available on the market for the disease to be treated or prevented. In such cases, the compounds of the invention may be administered consecutively, simultaneously or sequentially with the one or more other active agents.

In one embodiment of the invention, the peptide active agent may be administered in the form of a conjugate comprising the peptide, a linker, and an antibody molecule (or antibody fragment) intended to increase the half-life of the conjugate in-vivo.

"Modified peptides": In one embodiment, the peptides of the invention (including peptide variants) may be a modified peptide. The term "modified peptide" is used interchangeably with the term derivative of the peptide. In one embodiment, the term "modified peptide" means a peptide that is modified to exhibit one or more of the following properties compared with the unmodified peptide: increase plasma half-life; increase the lipophilicity of the peptide; increase the renal clearance of the modified peptide; and increase the resistance of the modified peptide to proteolytic degradation, while typically retaining the rpS6 phosphorylation activity. Various methods of modifying a peptide of the invention to exhibit these properties are disclosed herein, including conjugating the peptide with a binding partner (for example an albumin binding small molecule, large polymer, long life plasma protein, or antibody or antibody-fragment), cyclisation, addition of N- or C-terminal, or side chain, protecting groups, replacing L-amino acids with D-isomers, amino acid modification, increased plasma protein binding, increased albumin binding The modified peptide includes but is not limited to a peptide which has been substituted with one or more groups as defined herein, or conjugated with a binding partner, or cyclized. Generally, the peptide is modified to increase it half-life in-vivo in an animal. Various methods of modification are provided below.

In one embodiment, the modification may be any modification that provides the peptides and or the composition of the invention with an increased ability to penetrate a cell. In one embodiment, the modification may be any modification that increases the half-life of the composition or peptides of the invention. In one embodiment, the modification may be any modification that increases activity of the composition or peptides of the invention. In one embodiment, the modification may be any modification that increases selectivity of the composition or peptides of the invention.

In one embodiment, the group is a protecting group. The protecting group may be an N-terminal protecting group, a C-terminal protecting group or a side-chain protecting group. The peptide may have one or more of these protecting groups.

The person skilled in the art is aware of suitable techniques to react amino acids with these protecting groups. These groups can be added by preparation methods known in the art, for example the methods as outlined in paragraphs [0104] to [0107] of US2014120141. The groups may remain on the peptide or may be removed. The protecting group may be added during synthesis.

In an embodiment of the invention the peptides may be substituted with a group selected from one or more straight chain or branched chain, long or short chain, saturated, or unsaturated, substituted with a hydroxyl, amino, amino acyl, sulfate or sulphide group or unsubstituted having from 1 to 29 carbon atoms. N-acyl derivatives include acyl groups derived from acetic acid, capric acid, lauric acid, myristic acid, octanoic acid, palmitic acid, stearic acid, behenic acid, linoleic acid, linolenic acid, lipoic acid, oleic acid, isosteric acid, elaidoic acid, 2-ethylhexaneic acid, coconut oil fatty acid, tallow fatty acid, hardened tallow fatty acid, palm kernel fatty acid, lanolin fatty acid or similar acids. These may be substituted or unsubstituted. When substituted they are preferably substituted with hydroxyl, or sulphur containing groups such as but not limited to SO3H, SH, or S-S.

In an embodiment of the current invention, the peptide is R1-X- R2.

R1 and/or R2 groups respectively bound to the amino-terminal (N-terminal) and carboxyl-terminal (C-terminal) of the peptide sequence.

In one embodiment, the peptide is R1-X. Alternatively, the peptide is X- R2.

Preferably, R1 is H, C1-4 alkyl, acetyl, benzoyl or trifluoroacetyl;
X is the peptide of the invention;
R2 is OH or NH2.

In an embodiment, R 1 is selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl, Tert-butyloxycarbonyl, 9-fluorenylmethyloxycarbonyl (Fmoc) and R5-CO-, wherein R5 is selected from the group formed by H, a non-cyclic substituted or unsubstituted aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl;

R2 is selected from the group formed by -NR3R4, -OR3 and -SR3, wherein R3 and R4 are independently selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and with the condition that R1 and R2 are not α-amino acids.

In accordance with another preferred embodiment, R2 is -NR3R4, -OR 3 or -SR 3 wherein R3 and R4 are independently selected from the group formed by H, substituted or unsubstituted C 1-C 24 alkyl, substituted or unsubstituted C2-C 24 alkenyl, Tert-butyloxycarbonyl, 9-fluorenylmethyloxycarbonyl (Fmoc), substituted or unsubstituted C2-C 24 alkynyl, substituted or unsubstituted C3-C 24 cycloalkyl, substituted or unsubstituted C 5-C 24 cycloalkenyl, substituted or unsubstituted C8-C 24 cycloalkynyl, substituted or unsubstituted C 6-C 30 aryl, substituted or unsubstituted C7-C24 aralkyl, substituted or unsubstituted heterocyclyl ring of 3-10 members, and substituted or unsubstituted heteroarylalkyl of 2 to 24 carbon atoms and 1 to 3 atoms other than carbon wherein the alkyl chain is of 1 to 6 carbon atoms. Optionally, R 3 and R 4 can be bound by a saturated or unsaturated carbon-carbon bond, forming a cycle with the nitrogen atom. More preferably R 2 is -NR3R4 or -OR 3, wherein R3 and R4 are independently selected from the group formed by H, substituted or unsubstituted C1-C 24 alkyl, substituted or unsubstituted C2-C24 alkenyl, substituted or unsubstituted C2-C24 alkynyl, substituted or unsubstituted C3-C10 cycloalkyl, substituted or unsubstituted C6-C 15 aryl and substituted or unsubstituted heterocyclyl of 3-10 members, substituted or unsubstituted heteroarylalkyl with a ring of 3 to 10 members and an alkyl chain of 1 to 6 carbon atoms. More preferably R3 and R4 are selected from the group formed by H, methyl, ethyl, hexyl, dodecyl, or hexadecyl. Even more preferably R3 is H and R4 is selected from the group formed by H, methyl, ethyl, hexyl, dodecyl, or hexadecyl. In accordance with an even more preferred embodiment, R2 is selected from -OH and -NH2.

In accordance with another embodiment of this invention R 1 is selected from the group formed by H, acetyl, lauroyl, myristoyl or palmitoyl, and R2 is -NR3R 4 or -OR3 wherein R3 and R4 are independently selected from H, methyl, ethyl, hexyl, dodecyl and hexadecyl, preferably R2 is -OH or -NH2. More preferably, R1 is acetyl or palmitoyl and R2 is -NH2.

In a preferred embodiment, the acyl group is bound to the N-terminal end of at least one amino acid of the peptide.

In an embodiment of the invention, the peptide is modified to comprise a side chain protecting group. The side chain protecting group may be one or more of the group comprising benzyl or benzyl based groups, t-butyl-based groups, benzyloxy-carbonyl (Z) group, and allyloxycarbonyl (alloc) protecting group. The side chain protecting group may be derived from an achiral amino acid such as achiral glycine. The use of an achiral amino acid helps to stabilise the resultant peptide and also facilitate the facile synthesis route of the present invention. Preferably, the peptide further comprises a modified C-terminus, preferably an amidated C-terminus. The achiral residue may be alpha-aminoisobutyric acid (methylalaine). It will be appreciated that the specific side chain protecting groups used will depend on the sequence of the peptide and the type of N-terminal protecting group used.

In one embodiment of the invention the peptide is conjugated, linked or fused to one or more polyethylene glycol polymers or other compounds, such as molecular weight increasing compounds. The molecular weight increasing compound is any compound that will increase the molecular weight, typically by 10% to 90%, or 20% to 50% of the resulting conjugate and may have a molecular weight of between 200 and 20, 000, preferably between 500 and 10, 000. The molecular weight increasing compound may be PEG, any water-soluble(amphiphilic or hydrophilic) polymer moiety, homo or co-polymers of PEG, a monomethyl-subsitututed polymer of PEG (mPEG) and polyoxyethylene glycerol (POG), polyamino acids such as poly-lysine, poly-glutamic acid, poly-aspartic acid, particular those of L conformation, pharmacologically inactive proteins such as albumin, gelatin, a fatty acid, olysaccharide, a lipid amino acid and dextran. The polymer moiety may be straight chained or branched and it may have a molecular weight of 500 to 40000Da, 5000 to 10000 Da, 10000 to 5000, Da. The compound may be any suitable cell penetrating compound, such as tat peptide, penetratin, pep-1. The compound may be an antibody molecule. The compound may be a lipophilic moiety or a polymeric moiety.

The lipophilic substituent and polymeric substituents are known in the art. The lipophilic substituent includes an acyl group, a sulphonyl group, an N atom, an O atom or an S atom which forms part of the ester, sulphonyl ester, thioester, amide or sulphonamide. The lipophilic moiety may include a hydrocarbon chain having 4 to 30 C atoms, preferably between 8 and 12 C atoms. It may be linear or branched, saturated or unsaturated. The hydrocarbon chain may be further substituted. It may be cycloalkane or heterocycloalkane.

The peptide may be modified at the N-terminal, C-terminal or both. The polymer or compound is preferably linked to an amino, carboxyl or thio group and may be linked by N-termini or C-termini of side chains of any amino acid residue. The polymer or compound may be conjugated to the side chain of any suitable residue.

The polymer or compound may be conjugated via a spacer. The spacer may be a natural or unnatural amino acid, succinic acid, lysyl, glutamyl, asparagyl, glycyl, beta-alanyl, gamma-amino butanoyl.The polymer or compound may be conjugated via an ester, a sulphonyl ester, a thioester, an amide, a carbamate, a urea, a sulphonamide.A person skilled in the art is aware of suitable means to prepare the described conjugate.

Peptides can be chemically modified by covalent conjugation to a polymer to increase their circulating half-life, for example. Exemplary polymers and methods to attach such polymers to peptides are illustrated in, e.g., U.S. Pat. Nos. 4,766,106; 4,179,337; 4,495,285; and 4,609,546. Additional illustrative polymers include polyoxyethylated polyols and polyethylene glycol (PEG) moieties.

The peptides of the invention may be subjected to one or more modifications for manipulating storage stability, pharmacokinetics, and/or any aspect of the bioactivity of the peptide, such as, e.g., potency, selectivity, and drug interaction. Chemical modification to which the peptides may be subjected includes, without limitation, the conjugation to a peptide of one or more of polyethylene glycol (PEG), monomethoxy-polyethylene glycol, dextran, poly-(N-vinyl pyrrolidone) polyethylene glycol, propylene glycol homopolymers, a polypropylene oxide/ethylene oxide co-polymer, polypropylene glycol, polyoxyethylated polyols (e.g., glycerol) and polyvinyl alcohol, colominic acids or other carbohydrate based polymers, polymers of amino acids, and biotin derivatives. PEG conjugation of proteins at Cys residues is disclosed, e.g., in Goodson, R. J. & Katre, N. V. (1990) Bio/Technology 8, 343 and Kogan, T. P. (1992) Synthetic Comm. 22, 2417.

Modified peptides also can include sequences in which one or more residues are modified (i.e., by phosphorylation, sulfation, acylation, PEGylation, etc.), and mutants comprising one or more modified residues with respect to a parent sequence. Amino acid sequences may also be modified with a label capable of providing a detectable signal, either directly or indirectly, including, but not limited to, radioisotope, fluorescent, and enzyme labels. Fluorescent labels include, for example, Cy3, Cy5, Alexa, BODIPY, fluorescein (e.g., FluorX, DTAF, and FITC), rhodamine (e.g., TRITC), auramine, Texas Red, AMCA blue, and Lucifer Yellow. Preferred isotope labels include 3H, 14C, 32 P, 35S, 36CI, 51Cr, 57Co, 58Co, 59Fe, 90Y, 1251, 1311, and 286Re. Preferred enzyme labels include peroxidase, β-glucuronidase, β-D-glucosidase, β-D-galactosidase, urease, glucose oxidase plus peroxidase, and alkaline phosphatase (see, e.g., U.S. Pat. Nos. 3,654,090; 3,850,752 and 4,016,043). Enzymes can be conjugated by reaction with bridging molecules such as carbodiimides, diisocyanates, glutaraldehyde, and the like. Enzyme labels can be detected visually, or measured by calorimetric, spectrophotometric, fluorospectrophotometric, amperometric, or gasometric techniques. Other labeling systems, such as avidin/biotin, Tyramide Signal Amplification (TSA™), are known in the art, and are commercially available (see, e.g., ABC kit, Vector Laboratories, Inc., Burlingame, Calif.; NEN® Life Science Products, Inc., Boston, Mass.).

In an embodiment, the peptide, variant and/or composition is modified to increase drug performance ability. In an embodiment, the peptide, variant and/or composition is modified to increase stability, permeability, maintain potency, avoid toxicity and/or to increase half-life. The modification may be as described above. For example, the modification may be to protect the N and C-terminus, it may be a modified amino acid, cyclisation, replacement of an amino acid, and/or conjugation to macromolecules or large polymers or long life plasma proteins. Strategies to extend a half-life may be as described by Strohl, et al (BioDrugs, 2015), Schlapschy, et al (Protein Eng Des Sel. 2013), Podust, VN, et al (Protein Eng Des Sel. 2013), Zhang, L et al (Curr Med Chem. 2012), Gaberc-Porekar, V, et al (Curr Opin Drug Discov Devel. 2008). Examples include using PEGylation, lipidation (covalent binding of fatty acids to peptide side chains), fusion to Fc domains and human serum albumin, fusion with a hydrophilic amino acid polymer, e.g. XTEN or PAS, and/or fusion with half-life extension proteins.

Modification of peptides to extend the in-vivo half-life of the peptide is described in the literature, for example:
Strategies to improve plasma half life time of peptide and protein drugs. Werle M, Bernkop-Schnürch A. Amino Acids. 2006 Jun;30(4):351-67.

Due to the obvious advantages of long-acting peptide and protein drugs, strategies to prolong plasma half life time of such compounds are highly on demand. Short plasma half life times are commonly due to fast renal clearance as well as to enzymatic degradation occurring during systemic circulation. Modifications of the peptide/protein can lead to prolonged plasma half life times. By shortening the overall amino acid amount of somatostatin and replacing L: - analogue amino acids with D: -amino acids, plasma half life time of the derivate octreotide was 1.5 hours in comparison to only few minutes of somatostatin. A PEG(2,40 K) conjugate of INF-alpha-2b exhibited a 330-fold prolonged plasma half life time compared to the native protein. It was the aim of this review to provide an overview of possible strategies to prolong plasma half life time such as modification of N- and C-terminus or PEGylation as well as methods to evaluate the effectiveness of drug modifications. Furthermore, fundamental data about most important proteolytic enzymes of human blood, liver and kidney as well as their cleavage specificity and inhibitors for them are provided in order to predict enzymatic cleavage of peptide and protein drugs during systemic circulation.

Strategic Approaches to Optimizing Peptide ADME Properties. Li Di AAPS J. 2015 Jan; 17(1): 134-143.

### Strategies to Stabilize Peptides from Proteolysis

Many approaches are available to enhance stability of peptides through structure modification. Some approaches not only improve stability, but also enhance other ADME properties, e.g., cyclization can increase stability and permeability; conjugation to macromolecules can improve stability and reduce renal clearance. It is important to maintain potency and avoid toxicity while improving stability and ADME properties of peptides.

### • Protecting N- and C-terminus

A number of proteolytic enzymes in blood/plasma, liver or kidney are exopeptidases, aminopeptidases and carboxypeptidases and they break down peptide sequences from the N- and C-termini. Modification of the N- or/and C-termini can often improve peptide stability. Many examples have reported that N-acetylation, and C-amidation increase resistance to proteolysis.

### • Replacing L-amino acids with D-amino acids

Substituting natural L-amino acids with nonnatural D-amino acids decreases the substrate recognition and binding affinity of proteolytic enzymes and increases stability. One example is vasopressin, which contains an L-Arg and has a half-life of 10-35 min in humans. The D-Arg analog, desmopressin, has a half-life of 3.7 h in healthy human volunteers. In the study of a bicyclic peptide inhibitor of the cancer-related protease urokinase-type plasminogen activator (uPA), replacement of a specific glycine with a D-serine not only improves potency by 1.8-fold but also increases stability by 4-fold in mouse plasma.

### • Modification of amino acids

Modification of natural amino acids can improve the stability of peptides by introducing steric hindrance or disrupting enzyme recognition . For example, gonadotropin-releasing hormone has a very short half-life (minutes), while buserelin, in which one Gly is replaced with a t-butyl-D-Ser and another Gly is substituted by ethylamide, has a much longer half-life in humans.

### • Cyclization

The peptide of the invention may be cyclised. Cyclization introduces conformation constraint, reduces the flexibility of peptides, and increases stability and permeability. Depending on the functional groups, peptides can be cyclized head-to-tail, head/tail-to-side-chain, or side-chain-to-side-chain. Cyclization is commonly accomplished through lactamization, lactonization, and sulfide-based bridges. Disulfide bridges create folding and conformational constraints that can improve potency, selectivity, and stability. A number of disulfide bond-rich peptides are on the market or in preclinical or clinical development, e.g., linaclotide, lepirudin, and ziconotide.ln one embodiment, the peptide is cyclised between between amino and carboxy ends of the peptide. In one embodiment, the peptide is cyclised between an amino end and a side chain. In one embodiment, the peptide is cyclised between a carboxy end and a side chain. In one embodiment, the peptide is cyclised between side chains. In one embodiment, the cyclic peptide is selected from a homodetic cyclic peptide, a cyclic isopeptide, a cyclic depsipeptide, or a monocyclic or bicyclic peptide. Methods of cyclisation of peptides are described in the following:
Jensen, Knud (2009-09-01). Peptide and Protein Design for Biopharmaceutical Applications. John Wiley & Sons. ISBN 9780470749715.
Wenyan, Xu; Jun, Tang; Changjiu, Ji; Wenjun, He; Ninghua, Tan (2008). "Application of a TLC chemical method to detection of cyclotides in plants". Science Bulletin. 53 (11): 1671-1674. doi:10.1007/s11434-008-0178-8.
Borthwick AD (May 2012). "2,5-Diketopiperazines: Synthesis, Reactions, Medicinal Chemistry, and Bioactive Natural Products". Chemical Reviews. 112 (7): 3641-3716. doi:10.1021/cr200398y. PMID 22575049.
Barber, Carla J. S.; Pujara, Pareshkumar T.; Reed, Darwin W.; Chiwocha, Shiela; Zhang, Haixia; Covello, Patrick S. (2013). "The Two-step Biosynthesis of Cyclic Peptides from Linear Precursors in a Member of the Plant Family Caryophyllaceae Involves Cyclization by a Serine Protease-like Enzyme". Journal of Biological Chemistry. 288 (18): 12500-12510. doi:10.1074/jbc.M112.437947. PMC 3642298. PMID 23486480.
Wenyan Xu; et al. (2011). "Various mechanisms in cyclopeptide production from precursors synthesized independently of non-ribosomal peptide synthetases". Acta Biochimica et Biophysica Sinica. 43 (10): 757-762. doi:10.1093/abbs/gmr062. PMC 3180235. PMID 21764803.
Wenyan Xu; et al. "Plant Cyclopeptides and Possible Biosynthetic Mechanisms".
David J. Craik (17 March 2006). "Seamless Proteins Tie Up Their Loose Ends". Science. 311 (5767): 1563-7. doi:10.1126/science.1125248. PMID 16543448.

### • Conjugation to Macromolecules

Conjugation to macromolecules (e.g., polyethylene glycol (PEG), albumin) is an effective strategy to improve stability of peptides and reduce renal clearance.

### Renal Clearance

Many peptides exhibit promising in vitro pharmacological activity but fail to demonstrate in vivo efficacy due to very short in vivo half-life (minutes). The rapid clearance and short half-life of peptides hamper their development into successful drugs. The main causes of rapid clearance of peptides from systemic circulation are enzymatic proteolysis or/and renal clearance. The glomeruli have a pore size of ∼8 nm, and hydrophilic peptides with MW <2-25 kDa are susceptible to rapid filtration through the glomeruli of the kidney. Since peptides are not easily reabsorbed through the renal tubule, they frequently have high renal clearance and short half-life. Other minor routes of peptide clearance are endocytosis and degradation by proteasome and the liver. Comparison between systemic and renal clearance in animal models provides useful information on whether renal clearance is likely to be a major elimination pathway.

For renal-impaired patients, dose adjustment may be needed for peptide drugs to avoid accumulation and higher drug exposure, as inappropriate dosing in patients with renal dysfunction can cause toxicity or ineffective therapy. Several strategies have been developed to reduce peptide renal clearance and prolong half-life. These will be reviewed next.

### • Increase plasma protein binding

Renal clearance of peptides is reduced when they are bound to membrane proteins or serum proteins. An example is the cyclic peptide drug octreotide, a treatment for endocrine tumors, which has about 100 min half-life in humans due to binding to lipoproteins (fraction unbound 0.65)

### • Covalent Linkage to Albumin-Binding Small Molecules

Covalently attaching albumin-binding small molecules to peptides can reduce glomerular filtration, improve proteolytic stability, and prolong half-life by indirectly interacting with albumin through the highly bound small molecules.

### • Conjugation to Large Polymers

Conjugation of peptides to large synthetic or natural polymers or carbohydrates can increase their molecular weight and hydrodynamic volume, thus reducing their renal clearance. The common polymers used for peptide conjugation are PEG, polysialic acid (PSA), and hydroxyethyl starch (HES).

### • Fusion to Long-Live Plasma Proteins

Plasma proteins, such as albumin and immunoglobulin (IgG) fragments, have long half-lives of 19-21 days in humans. Because of the high MW (67-150 kDa), these proteins have low renal clearance, and their binding to neonatal Fc receptor (FcRn) reduces the elimination through pinocytosis by the vascular epithelium. Covalent linkage of peptides to albumin or IgG fragments can reduce renal clearance and prolong half-life.

Fusion Proteins for Half-Life Extension of Biologics as a Strategy to Make Biobetters
William R. Strohl BioDrugs. 2015; 29(4): 215-239.
Schlapschy, M, Binder, U, Borger, C et al. PASYlation: a biological alternative to PEGylation for extending the plasma half-life of pharmaceutically active proteins. Protein Eng Des Sel. 2013;26(8):489-501.
Podust, VN, Sim, BC, Kothari, D et al. Extension of in vivo half-life of biologically active peptides via chemical conjugation to XTEN protein polymer. Protein Eng Des Sel. 2013;26(11):743-53.
Zhang, L, Bulaj, G. Converting Peptides into Drug Leads by Lipidation. Curr Med Chem. 2012;19(11):1602-18.
Gaberc-Porekar, V, Zore, I, Podobnik, B et al. Obstacles and pitfalls in the PEGylation of therapeutic proteins. Curr Opin Drug Discov Devel. 2008;11(2):242-50.
By Dr Ronald V. Swanson - Long live peptides evolution of peptide half-life extension technologies and emerging hybrid approaches. From Drug Discovery World on line.

### Spring 2014

### PEGylation

The attachment of long chains of the hydrophilic polymer polyethylene glycol to molecules of interest, PEGylation was originally conceived as a modification to prevent the recognition of foreign proteins by the immune system and, thereby, enable their utility as therapeutics. Once formed, antibodies against unmodified drugs can rapidly neutralise and clear protein drugs. Unexpectedly, PEGylation improved the pharmacokinetics of the proteins even in the absence of anti-drug antibodies1. Simply by making drug molecules larger, PEGylation led to the drug being filtered more slowly by the kidneys. The empirical observation that increasing size or hydrodynamic radius led to reduced renal clearance and increased half-life then became the dominant rationale for the PEGylation of protein and peptide drugs. PEGylation can have a variety of effects on the molecule including making proteins or peptides more water-soluble and protecting them from degradation by proteolytic enzymes. PEGylation can also impact the binding of therapeutic proteins to their cognate cellular receptors, usually reducing the affinity. Changes in the size, structure and attachment mode of PEG polymers can affect the biological activity of the attached drug.

The first-generation PEGylation methods were filled with challenges. However, the chemistry of PEGylation is quite simple. The process involves the covalent attachment of polyethylene glycol chains to reactive side chains of a protein or peptide. For example, PEG is easily attached to the -amino groups of lysine on the surface of proteins or peptides2. The reaction is pH-dependent. At high pH (8.0 or higher), lysine side chain amino groups are covalently attached to PEG through N-hydroxy succinimides. This method typically results in a family of products containing different numbers of PEG chains attached at different sites on a protein rather than a single discrete product3. The first approved PEGylated pharmaceuticals were Pegademase bovine (PEGylated bovine adenosine deamidase) as enzyme replacement therapy for severe combined immunodeficiency and Pegaspargase (PEGylated asparaginase) for treatment of acute lymphoblastic leukaemia1. These drugs were complex mixtures of various PEGylated species, but with improved properties for therapy over native enzymes, including increased serum half-life and decreased immunogenicity of the proteins. Due to the inherent polydispersity of the PEG, quality and batch-to-batch reproducibility was difficult. Despite this limitation, two PEGylated interferons, (Peginterferon alfa-2b and Peginterferon alfa-2a) that are heterogeneous populations of numerous mono-PEGylated positional isomers, have been FDA-approved for the treatment of hepatitis C. These drugs were brought to market in 2001 and 2002, respectively.

A variety of enhancements and variations have been made to the fundamental PEGylation technology. Second-generation PEGylation processes introduced the use of branched structures as well as alternative chemistries for PEG attachment. In particular, PEGs with cysteine reactive groups such as maleimide or iodoacetamide allow the targeting of the PEGylation to a single residue within a peptide or protein reducing the heterogeneity of the final product but not eliminating it due to the polydispersity of the PEG itself.

While the original rationale for PEGylation was to reduce immunogenicity; nevertheless, there have been a few examples of immunogenic PEGylated proteins. One example is PEGylated urate oxidase, an enzyme that lowers the plasma urate level in patients with gout. In clinical trials, a relatively high percentage of patients with gout did not respond to the therapy and developed antibodies that were specific for PEG, but not for the uricase protein2. PEGylated liposomes, also generally thought to be non-immunogenic, have been found to be immunogenic in some studies. PEGylated liposomes elicit a strong anti-PEG immunoglobulin M (IgM) response. In addition, multiple injections of PEG-glucuronidase were shown to elicit the generation of specific anti-PEG IgM antibodies, thus accelerating the clearance of PEG-modified proteins from the body.

A major potential drawback of using PEG as a modifier is that it is non-biodegradable. The US Food and Drug Administration (FDA) has approved PEG for use as a vehicle in pharmaceuticals, including injectable, topical, rectal and nasal formulations. PEG shows little toxicity and is eliminated from the body intact by either the kidneys (for PEGs < 30 kDa) or in the feces (for PEGs >20 kDa)1. Repeated administration of some PEGylated proteins to animals has resulted in observations of renal tubular cellular vacuolation. Recently, vacuolation of choroid plexus epithelial cells has also been seen in toxicity studies with proteins conjugated with large (≥40 kDa) PEGs. The choroid plexus epithelial cells produce cerebrospinal fluid and form the blood CSF barrier. The long-term negative consequences of cellular vacuolation are unclear, but it does represent an undesirable consequence for some potential therapeutics. One possible alternative would be substitution of a biodegradable polymer in place of PEG. Polymers, such as hydroxyethyl starch (HES) are a possible alternative. HES is non-toxic and biodegradable and used as a blood expander. A process of HESylation would function similarly to PEGylation in reducing renal clearance through increasing a peptide's hydrodynamic radius but may confer a lower propensity for accumulation due to biodegradability. However, HES and other proposed biodegradable polymer PEG alternatives are, like PEG, polydisperse making characterisation of the final product and metabolites difficult. One emerging solution which mitigates both concerns is to use defined polypeptides as the polymer component; this approach will be discussed later in the article.

### Lipidation

A second major chemical modification method to increase peptide half-life is lipidation which involves the covalent binding of fatty acids to peptide side chains4. Originally conceived of and developed as a method for extending the half-life of insulin, lipidation shares the same basic mechanism of half-life extension as PEGylation, namely increasing the hydrodynamic radius to reduce renal filtration. However, the lipid moiety is itself relatively small and the effect is mediated indirectly through the non-covalent binding of the lipid moiety to circulating albumin. A large (67 KDa) and highly abundant protein in human serum (35- 50g/L), albumin naturally functions to transport molecules, including lipids, throughout the body. Binding to plasma proteins can also protect the peptide from attacks by peptidases through steric hindrance, again akin to what is seen with PEGylation. One consequence of lipidation is that it reduces the water-solubility of the peptide but engineering of the linker between the peptide and the fatty acid can modulate this, for example by the use of glutamate or mini PEGs within the linker. Linker engineering and variation of the lipid moiety can affect self-aggregation which can contribute to increased half-life by slowing down biodistribution, independent of albumin5.

Following the pioneering work with insulin6, lipidation of a variety of peptides has been explored, particularly peptides within the diabetes space including human glucagon-like peptide-1 (GLP-1) analogues, glucose-dependent insulinotropic polypeptide and GLP-1 R/Glucagon receptor coagonists among others. Two lipidated peptide drugs are currently FDA-approved for use in humans. These are both long-acting anti-diabetics, the GLP- 1 analogue liraglutide and insulin detemir.

A potentially pharmacologically-relevant difference between PEGylation and lipidation is that the therapeutically active peptide is covalently linked to the much larger PEG, whereas the smaller fatty acyl-peptide conjugate is non-covalently associated with the larger albumin, bound and unbound forms existing in equilibrium. This can result in differences in biodistribution that may result in different pharmacology as access to receptors localised in different tissues may elicit differential effects. In some cases, more restricted biodistribution may be desirable, while in others, greater tissue penetration may be important. An interesting variation of the PEG approach which addresses this issue has been developed by Santi et al in which releasable PEG conjugates with predictable cleavage rates are utilised7.

PEGylation and lipidation both confer protection against proteases and peptidases by shielding through steric hindrance and extend circulating half-life through increased hydrodynamic radius, directly or indirectly. Both methods utilise chemical conjugation and are flexible in that they are agnostic to the means used to generate the peptide they are modifying, whether biologically or synthetically produced. An advantage of using synthetic peptides is that they can incorporate non-natural amino acids designed to address a number of specific issues including instability due to known proteolytic cleavage liabilities. They can also be more flexible in terms of the choice of attachment site which is critical if activity or potency is highly dependent on the free termini or a modified residue such as a C terminal amide.

### Classical genetic fusions: Fc and HSA

Classical genetic fusions to long-lived serum proteins offer an alternative method of half-life extension distinct from chemical conjugation to PEG or lipids. Two major proteins have traditionally been used as fusion partners: antibody Fc domains and human serum albumin (HAS). Fc fusions involve the fusion of peptides, proteins or receptor exodomains to the Fc portion of an antibody. Both Fc and albumin fusions achieve extended half-lives not only by increasing the size of the peptide drug, but both also take advantage of the body's natural recycling mechanism: the neonatal Fc receptor, FcRn. The pH-dependent binding of these proteins to FcRn prevents degradation of the fusion protein in the endosome. Fusions based on these proteins can have half-lives in the range of 3-16 days, much longer than typical PEGylated or lipidated peptides. Fusion to antibody Fc can improve the solubility and stability of the peptide or protein drug. An example of a peptide Fc fusion is dulaglutide, a GLP-1 receptor agonist currently in late-stage clinical trials. Human serum albumin, the same protein exploited by the fatty acylated peptides is the other popular fusion partner. Albiglutide is a GLP-1 receptor agonist based on this platform. A major difference between Fc and albumin is the dimeric nature of Fc versus the monomeric structure of HAS leading to presentation of a fused peptide as a dimer or a monomer depending on the choice of fusion partner. The dimeric nature of a peptide Fc fusion can produce an avidity effect if the target receptors are spaced closely enough together or are themselves dimers. This may be desirable or not depending on the target.

### Designed polypeptide fusions: XTEN and PAS

An intriguing variation of the recombinant fusion concept has been the development of designed low complexity sequences as fusion partners, basically unstructured, hydrophilic amino acid polymers that are functional analogs of PEG. The inherent biodegradability of the polypeptide platform makes it attractive as a potentially more benign alternative to PEG. Another advantage is the precise molecular structure of the recombinant molecule in contrast to the polydispersity of PEG. Unlike HSA and Fc peptide fusions, in which the three-dimensional folding of the fusion partner needs to be maintained, the recombinant fusions to unstructured partners can, in many cases, be subjected to higher temperatures or harsh conditions such as HPLC purification.

The most advanced of this class of polypeptides is termed XTEN (Amunix) and is 864 amino acids long and comprised of six amino acids (A, E, G, P, S and T). Enabled by the biodegradable nature of the polymer, this is much larger than the 40 KDa PEGs typically used and confers a concomitantly greater half-life extension. The fusion of XTEN to peptide drugs results in half-life extension by 60- to 130-fold over native molecules. Two fully recombinantly produced XTENylated products have entered the clinic, namely VRS-859 (Exenatide-XTEN) and VRS- 317 (human growth hormone-XTEN). In Phase la studies, VRS-859 was found to be well-tolerated and efficacious in patients with Type 2 diabetes. VRS-317 reported superior pharmacokinetic and pharmacodynamic properties compared with previously studied rhGH products and has the potential for once-monthly dosing.

A second polymer based on similar conceptual considerations is PAS (XL-Protein GmbH)9. A random coil polymer comprised of an even more restricted set of only three small uncharged amino acids, proline, alanine and serine. Whether differences in the biophysical properties of PAS and the highly negatively charged XTEN may contribute to differences in biodistribution and/or in vivo activity is yet unknown but will be revealed as these polypeptides are incorporated into more therapeutics and the behaviour of the fusions characterised.

All the peptide protein fusions, whether the partner is Fc, HSA, XTEN or PAS, are genetically encoded and consequently suffer from similar constraints. One limitation is that only naturally occurring amino acids are incorporated, unlike the methods employing chemical conjugation which allow the use of synthetic peptides incorporating non-natural amino acids. Although methods to overcome this by expanding the genetic code are being developed by companies such as Ambrx or Sutro, they are not yet in wide use. A second limitation is that either the Nor C-terminus of the peptide needs to be fused to the partner. Oftentimes, the peptide termini are involved in receptor interactions and genetic fusion to one or both termini can greatly impair activity. Since the site of PEG or lipid conjugation can be anywhere on the peptide, it can be optimised to maximise biological activity of the resulting therapeutic.

### Hybrid methods merging synthetic peptides with half-life extension proteins

While genetic fusions have historically offered the potential for greater half-life extension, they lack the advantages afforded by the methods utilising chemical conjugation, PEGylation and lipidation, in terms of flexibility of attachment sites and incorporation of unnatural amino acids or modifications to the peptide backbone. One of the first efforts to merge the advantages of the genetic fusions with chemical conjugation for half-life extension was carried out by researchers at the Scripps Research Institute in La Jolla with the technology which later formed the basis for the biotech company CovX10,11. Using a catalytic aldolase antibody, these researchers developed a platform through which the active site lysine of the antibody forms a reversible covalent enamine bond with a beta-diketone incorporated into a peptide or small molecule. The resulting complex is termed a CovXBody ™. This approach combines the functional qualities of a peptide drug or small molecule with the long serum half-life of an antibody, not through a genetic fusion but rather through a chemical linkage. Following the initial demonstration of the technology, researchers expanded upon the use of CovX-Body™ prototype that is based on an integrin targeting peptidomimetic pharmacophore. At least three molecules based on this architecture have entered clinical development: CVX-096, a Glp-1R agonist; CVX-060, an Angiopoietin-2 binding peptide; and CVX-045, a thrombospondin mimetic.

Recently, the XTEN polypeptide has also been used in a chemical conjugation mode making it even more directly analogous to PEG. The first example of an XTENylated peptide that was created using this method is GLP2-2G-XTEN in which the peptide is chemically conjugated to the XTEN protein polymer using maleimide-thiol chemistry. The chemically conjugated GLP2-2GXTEN molecules exhibited comparable in vitro activity, in vitro plasma stability and pharmacokinetics in rats comparable to recombinantly-fused GLP2-2G-XTEN.

The number and spacing of reactive groups such as lysine or cysteine side chains in the completely designed sequences of XTEN or PAS polypeptides can be precisely controlled through site-directed changes due to the restricted amino acid sets from which they are composed. This provides an additional degree of flexibility over methods which might utilise Fc or albumin whose sequences naturally contain many reactive groups and stands in contrast to the CovX technology which relies on a reactive residue in a highly specialised active site. In addition, the lack of tertiary structure of XTEN or PAS should provide more flexibility over the conditions and chemistries used in coupling and in the purification of conjugates.

In summary, hybrid peptide half-life extension methods are emerging that combine the advantages and overcome the individual limitations of chemical conjugation and genetic fusions methods. These methods enable the creation of molecules based on recombinant polypeptide-based partners that impart longer half-life but free the therapeutic peptide moieties from the limitations of being composed solely of natural L-amino acids or configured solely as linear, unidirectional polypeptides fused at either the N- or C-terminus, thus opening the door to a wide range of longer acting peptide-based drugs.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1

### Identification of peptide receptor/target protein

### Methodology

CY5-labelled peptide (SEQUENCE ID NO.1; (0.01 & 0.05 µg/ml) was screened for binding against fixed HEK293 Cells expressing 5528 human plasma membrane proteins. Screening was carried across two replicates and initial hits were selected and screened in more specific confirmation assay.

### Results

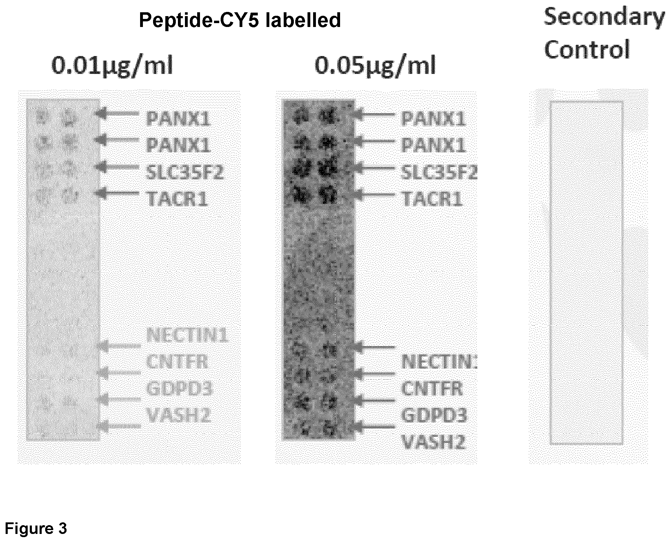

Figure 3 illustrates the binding affinity of the peptide of the invention to human membrane proteins. Three Highly Selective Targets were identified, Panx1, SLC35F2 & TACR1. Based on biology, tissue distribution and peptide binding to multiple Isoforms, it was concluded that PANX1 is the primary target for the peptide.

### EXAMPLE 2

### Peptide of the invention reduces secretion of IL-8 in immortal hepatocytes.

### Methodology

HepG2 cells were treated with peptide (5 ng/ml) for 24 h before treating with 100 ng/mL of LPS for 24 h. Data presented are the mean ± SEM of at least two independent experiments. (*p<0.05 **p<0.01 ***p<0.001)

### Results

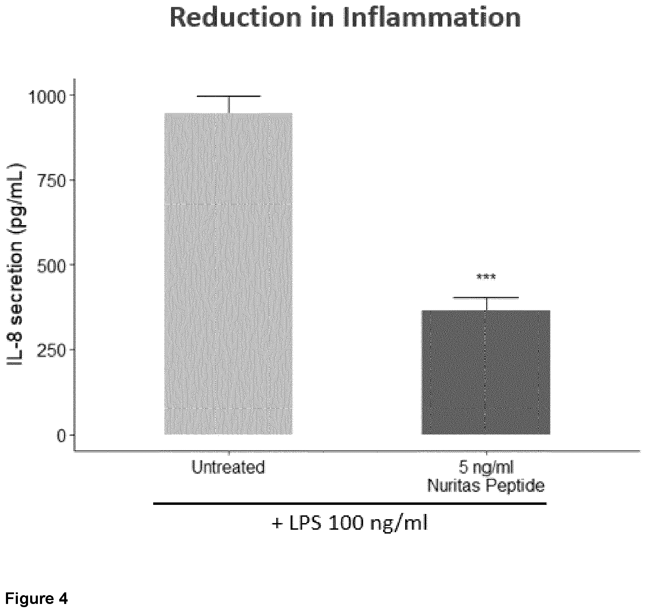

As illustrated in Figure 4 peptide of the invention reduced section of IL-8.

A hallmark of liver fibrosis is increased secretion of inflammatory marker such as IL-8. Reducing the expression of this marker demonstrates that the peptide has the ability to be efficacious in this disease setting.

### EXAMPLE 3

### Anti-fibrotic activity in human primary hepatic stellate cells.

### Methodology

Human stellate cells were treated with TGF-β1 to stimulate expression of α-SMA (smooth muscle action; primary marker for fibrosis) before treatment with the peptide (5 nM; SEQUENCE ID NO. 1). Confocal imaging of the cells was undertaken.

### Results

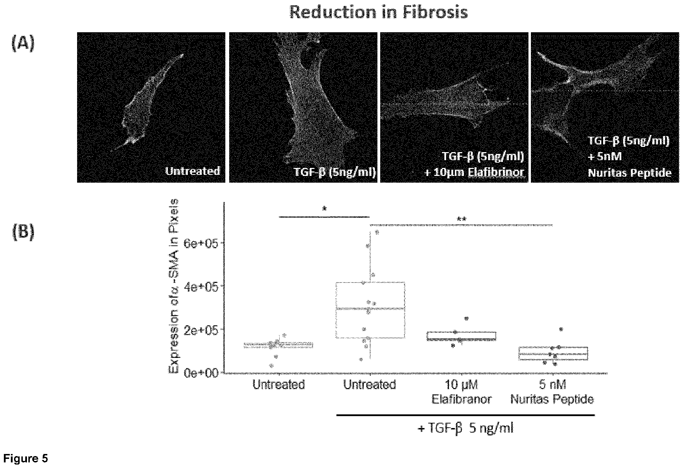

Figure 5 illustrates confocal imaging of the human stellate cells before and after treatment. The decrease in α-SMA expression is evidence of anti-fibrotic activity of the peptide. The peptide outperformed Elafibranor at equivalent molar doses

### EXAMPLE 4

### Intracellular peptide concentration after PANX1 activation

### Methodology

The peptide of the invention (SEQUENCE ID NO.1) was added to cells (Caco2). The PANX1 channel was activated in cells by increasing extracellular calcium levels. The intracellular concentration of the peptide was measured before and after activation.

### Results

Under certain physiological conditions, i.e. increased extracellular ATP or calcium, the pannexin channel is activated and allows greater influx of signalling molecules. Figure 7 shows that the intracellular concentration of the peptide is increased when the channel is activated. This is evidence that the peptide will preferably interact with the channel under activated or disease conditions. The green dye in Figure 7 is a membrane dye while the red dye is the labelled peptide.

## Claims

1. A peptide having up to 50 amino acids and comprising SEQUENCE ID NO: 1, or a therapeutically effective variant of SEQUENCE ID NO: 1, for use in a method for the treatment or prevention of a disease or condition associated with PANX1, in a subject, in which the disease or condition is selected from the group comprising fibrosis, melanoma, liver cancer, ischemia, hypertension, and multiple sclerosis.

2. The peptide for use of Claim 1, in which the fibrosis is selected from the group comprising hepatic fibrosis, skin fibrosis, renal fibrosis, pulmonary fibrosis,

3. The peptide for use of Claim 1 or 2, in which the peptide consists of SEQUENCE ID NO. 1.

4. The peptide for use of any one of the preceding claims, in which the therapeutically effective variant comprises 1 to 8 modifications compared with SEQUENCE ID NO: 1, in which the or each modification is selected from an insertion, addition, deletion and substitution of an amino acid.

5. The peptide for use of any one of the preceding claims, in which the therapeutically effective variant comprises 1 to 5 modifications compared with SEQUENCE ID NO: 1, in which the or each modification is selected from an insertion, addition, deletion and substitution of an amino acid.

6. The peptide for use of Claim 5, in which the therapeutically effective variant comprises 1 to 2 modifications compared with SEQUENCE ID NO: 1, in which the or each modification is selected from an insertion, addition, deletion and substitution of an amino acid.

7. The peptide for use of Claim 5 or 6, in which the modifications comprise substituting one or more of the amino acids of SEQUENCE ID NO: 1 with D-forms of the amino acids.

8. The peptide for use of Claim 7, in which the modifications comprise substituting at least two residues selected from resides 1, 2, 5, 10 and 11 of SEQUENCE ID NO: 1 with a D-form of the amino acid.

9. The peptide for use of any one of claims 7 or 8, in which the modifications comprise substituting at least three or four residues selected from resides 1, 2, 5, 10 and 11 of SEQUENCE ID NO: 1 with a D-form of the amino acid.

10. The peptide for use of any one of the preceding claims, in which the peptide variant is SEQUENCE ID NO. 86 or SEQUENCE ID NO. 87.

11. The peptide for use of any one of the preceding claims in which the peptide is modified.

12. The peptide for use of Claim 11, in which the peptide is modified by a modification(s) to side chains, incorporation of unnatural amino acids and/or their derivatives during peptide synthesis, the use of cross-linkers and other methods that impose conformational constraint on the peptide, by conjugation to a conjugation partner, by fusion to a fusion partner, covalent linkage to a binding partner, lipidation, PEGylation, and amidation.

13. The peptide for use of any one of the preceding claims, in which the peptide is cyclised.

14. A composition comprising a peptide having up to 50 amino acids and comprising SEQUENCE ID NO: 1, or a therapeutically effective variant of SEQUENCE ID NO: 1, for use in a method for the treatment or prevention of a disease or condition associated with PANX1, in a subject, in which the disease or condition is selected from the group comprising fibrosis, melanoma, liver cancer, ischemia, hypertension, and multiple sclerosis..


SEQUENCE LISTING

<110>  Nuritas

<120>  Treatment of PANX1 associated diseases

<130>  P13085EP01

<140>
<141>

<160>  89

<170>  PatentIn version 3.5

<210>  1
<211>  11
<212>  PRT
<213>  Artificial Sequence

<220>
<223>  Peptide

<400>  1

Trp Lys Asp Glu Ala Gly Lys Pro Leu Val Lys
1               5                   10


<210>  2
<211>  11
<212>  PRT
<213>  Artificial Sequence

<220>
<223>  Peptide

<400>  2

Trp Lys Glu Glu Ala Gly Lys Pro Leu Val Lys
1               5                   10


<210>  3
<211>  11
<212>  PRT
<213>  Artificial Sequence

<220>
<223>  Peptide

<400>  3

Phe Lys Asp Glu Ala Gly Lys Pro Leu Val Lys
1               5                   10


<210>  4
<211>  11
<212>  PRT
<213>  Artificial Sequence

<220>

<223>  Peptide

<400>  4

Trp Lys Asp Glu Ala Gly Lys Pro Met Val Lys
1               5                   10


<210>  5
<211>  11
<212>  PRT
<213>  Artificial Sequence

<220>
<223>  Peptide

<400>  5

Trp Lys Asp Glu Ala Gly Arg Pro Leu Val Lys
1               5                   10


<210>  6
<211>  11
<212>  PRT
<213>  Artificial Sequence

<220>
<223>  Peptide

<400>  6

Trp Arg Asp Glu Ala Gly Lys Pro Leu Val Lys
1               5                   10


<210>  7
<211>  11
<212>  PRT
<213>  Artificial Sequence

<220>
<223>  Peptide

<400>  7

Trp Lys Asp Glu Ala Gly Lys Pro Leu Met Lys
1               5                   10


<210>  8
<211>  11
<212>  PRT
<213>  Artificial Sequence

<220>
<223>  Peptide

<400>  8

Trp Lys Asp Gln Ala Gly Lys Pro Leu Val Lys
1               5                   10

<210>  9
<211>  11
<212>  PRT
<213>  Artificial Sequence

<220>
<223>  Peptide

<400>  9

Trp Lys Asp Glu Ala Thr Lys Pro Leu Val Lys
1               5                   10


<210>  10
<211>  11
<212>  PRT
<213>  Artificial Sequence

<220>
<223>  Peptide

<400>  10

Tyr Lys Asn Glu Ala Gly Lys Pro Leu Val Lys
1               5                   10


<210>  11
<211>  11
<212>  PRT
<213>  Artificial Sequence

<220>
<223>  Peptide

<400>  11

Trp Lys Asn Glu Ser Gly Lys Pro Leu Val Lys
1               5                   10


<210>  12
<211>  11
<212>  PRT
<213>  Artificial Sequence

<220>
<223>  Peptide

<400>  12

Trp Lys Asp Glu Ala Gly Lys Thr Leu Val Arg
1               5                   10


<210>  13
<211>  11
<212>  PRT
<213>  Artificial Sequence

<220>

<223>  Peptide

<400>  13

Phe Lys Asp Glu Ala Thr Lys Pro Leu Val Lys
1               5                   10


<210>  14
<211>  11
<212>  PRT
<213>  Artificial Sequence

<220>
<223>  Peptide

<400>  14

Phe Lys Asp Glu Ala Gly Lys Pro Leu Ile Lys
1               5                   10


<210>  15
<211>  11
<212>  PRT
<213>  Artificial Sequence

<220>
<223>  Peptide

<400>  15

Trp Lys Asp Glu Ala Gly Lys Thr Leu Leu Lys
1               5                   10


<210>  16
<211>  11
<212>  PRT
<213>  Artificial Sequence

<220>
<223>  Peptide

<400>  16

Trp Lys Asn Glu Ala Gly Lys Pro Val Val Lys
1               5                   10


<210>  17
<211>  11
<212>  PRT
<213>  Artificial Sequence

<220>
<223>  Peptide

<400>  17

Trp Lys Asp Glu Ala Gly Arg Thr Leu Val Lys
1               5                   10

<210>  18
<211>  11
<212>  PRT
<213>  Artificial Sequence

<220>
<223>  Peptide

<400>  18

Trp Glu Asp Glu Ser Gly Lys Pro Leu Leu Lys
1               5                   10


<210>  19
<211>  11
<212>  PRT
<213>  Artificial Sequence

<220>
<223>  Peptide

<400>  19

Trp Lys Glu Glu Ala Gly Lys Pro Ile Val Gln
1               5                   10


<210>  20
<211>  11
<212>  PRT
<213>  Artificial Sequence

<220>
<223>  Peptide

<400>  20

Tyr Lys Asn Glu Ala Gly Lys Pro Leu Val Arg
1               5                   10


<210>  21
<211>  11
<212>  PRT
<213>  Artificial Sequence

<220>
<223>  Peptide

<400>  21

Trp Lys Asp Gln Ala Thr Arg Pro Leu Val Lys
1               5                   10


<210>  22
<211>  11
<212>  PRT
<213>  Artificial Sequence

<220>

<223>  Peptide

<400>  22

Trp Lys Asp Glu Ser Gly Lys Pro Val Leu Lys
1               5                   10


<210>  23
<211>  11
<212>  PRT
<213>  Artificial Sequence

<220>
<223>  Peptide

<400>  23

Trp Gln Asp Asp Ser Gly Lys Pro Leu Val Lys
1               5                   10


<210>  24
<211>  11
<212>  PRT
<213>  Artificial Sequence

<220>
<223>  Peptide

<400>  24

Trp Lys Asn Glu Ala Gly Lys Thr Leu Leu Lys
1               5                   10


<210>  25
<211>  11
<212>  PRT
<213>  Artificial Sequence

<220>
<223>  Peptide

<400>  25

Trp Lys Asp Lys Ala Gly Glu Pro Leu Val Arg
1               5                   10


<210>  26
<211>  11
<212>  PRT
<213>  Artificial Sequence

<220>
<223>  Peptide

<400>  26

Trp Lys Asp Glu Ala Gly Asn Pro Leu Val Lys
1               5                   10

<210>  27
<211>  11
<212>  PRT
<213>  Artificial Sequence

<220>
<223>  Peptide

<400>  27

Cys Lys Asp Glu Ala Gly Lys Pro Leu Val Lys
1               5                   10


<210>  28
<211>  11
<212>  PRT
<213>  Artificial Sequence

<220>
<223>  Peptide

<400>  28

Trp Lys Asp Glu Ala Gly Lys Pro Leu Gly Lys
1               5                   10


<210>  29
<211>  11
<212>  PRT
<213>  Artificial Sequence

<220>
<223>  Peptide

<400>  29

Trp Lys Asp Glu Asn Gly Lys Pro Leu Val Lys
1               5                   10


<210>  30
<211>  11
<212>  PRT
<213>  Artificial Sequence

<220>
<223>  Peptide

<400>  30

Trp Lys Asp Glu Ala Arg Lys Pro Leu Val Lys
1               5                   10


<210>  31
<211>  11
<212>  PRT
<213>  Artificial Sequence

<220>

<223>  Peptide

<400>  31

Trp Lys Asp Glu Ala Gly Lys Pro Leu Val Thr
1               5                   10


<210>  32
<211>  11
<212>  PRT
<213>  Artificial Sequence

<220>
<223>  Peptide

<400>  32

Trp Lys Asp Glu Ala Gly Lys Arg Leu Val Lys
1               5                   10


<210>  33
<211>  11
<212>  PRT
<213>  Artificial Sequence

<220>
<223>  Peptide

<400>  33

Trp Lys Trp Glu Ala Gly Lys Pro Leu Val Lys
1               5                   10


<210>  34
<211>  11
<212>  PRT
<213>  Artificial Sequence

<220>
<223>  Peptide

<400>  34

Trp Lys Asp Glu Ala Gly Phe Pro Thr Val Lys
1               5                   10


<210>  35
<211>  11
<212>  PRT
<213>  Artificial Sequence

<220>
<223>  Peptide

<400>  35

Trp Tyr Asp Met Ala Gly Lys Pro Leu Val Lys
1               5                   10

<210>  36
<211>  11
<212>  PRT
<213>  Artificial Sequence

<220>
<223>  Peptide

<400>  36

Trp Lys Asp Tyr Glu Gly Lys Pro Leu Val Lys
1               5                   10


<210>  37
<211>  11
<212>  PRT
<213>  Artificial Sequence

<220>
<223>  Peptide

<400>  37

Trp Lys Arg Glu Ala Gly Lys Pro Gly Val Lys
1               5                   10


<210>  38
<211>  11
<212>  PRT
<213>  Artificial Sequence

<220>
<223>  Peptide

<400>  38

Trp Lys Leu Glu Lys Gly Lys Pro Leu Val Lys
1               5                   10


<210>  39
<211>  11
<212>  PRT
<213>  Artificial Sequence

<220>
<223>  Peptide

<400>  39

Trp Lys Asp Glu Ala Gly Lys Pro Cys Val Lys
1               5                   10


<210>  40
<211>  11
<212>  PRT
<213>  Artificial Sequence

<220>

<223>  Peptide

<400>  40

Trp Lys Lys Glu Ala Pro Lys Pro Leu Val Lys
1               5                   10


<210>  41
<211>  11
<212>  PRT
<213>  Artificial Sequence

<220>
<223>  Peptide

<400>  41

Ser Lys Asp Glu Ala Gly Pro Pro Leu Val Lys
1               5                   10


<210>  42
<211>  11
<212>  PRT
<213>  Artificial Sequence

<220>
<223>  Peptide

<400>  42

Trp Lys His Glu Pro Gly Lys Pro Leu Ala Lys
1               5                   10


<210>  43
<211>  11
<212>  PRT
<213>  Artificial Sequence

<220>
<223>  Peptide

<400>  43

Trp Lys Asp Glu Arg Glu Lys Pro Phe Val Lys
1               5                   10


<210>  44
<211>  11
<212>  PRT
<213>  Artificial Sequence

<220>
<223>  Peptide

<400>  44

Trp Lys Gln Glu Ala Gly Lys Pro Trp Arg Lys
1               5                   10

<210>  45
<211>  11
<212>  PRT
<213>  Artificial Sequence

<220>
<223>  Peptide

<400>  45

Val Lys Asp Glu Ala Lys Lys Pro Leu Val His
1               5                   10


<210>  46
<211>  11
<212>  PRT
<213>  Artificial Sequence

<220>
<223>  Peptide

<400>  46

Asn Trp Asp Glu Ala Gly Lys Met Leu Val Lys
1               5                   10


<210>  47
<211>  11
<212>  PRT
<213>  Artificial Sequence

<220>
<223>  Peptide

<400>  47

Ile Lys Asp Glu Asp Gly Pro Pro Leu Val Lys
1               5                   10


<210>  48
<211>  11
<212>  PRT
<213>  Artificial Sequence

<220>
<223>  Peptide

<400>  48

Leu Lys Asp Glu Tyr Gly Lys Pro Leu Val Asn
1               5                   10


<210>  49
<211>  11
<212>  PRT
<213>  Artificial Sequence

<220>

<223>  Peptide

<400>  49

Trp Lys Asp Arg Ala Gly Lys Glu Leu Thr Lys
1               5                   10


<210>  50
<211>  12
<212>  PRT
<213>  Artificial Sequence

<220>
<223>  Peptide

<400>  50

Trp Lys Asp Glu Ala Gly Lys Pro Leu Pro Val Lys
1               5                   10


<210>  51
<211>  14
<212>  PRT
<213>  Artificial Sequence

<220>
<223>  Peptide

<400>  51

Trp Lys Gly Asp Glu Asn Tyr Ala Gly Lys Pro Leu Val Lys
1               5                   10


<210>  52
<211>  14
<212>  PRT
<213>  Artificial Sequence

<220>
<223>  Peptide

<400>  52

Leu Trp Lys Asp Glu Ala Gly Arg Lys Tyr Pro Leu Val Lys
1               5                   10


<210>  53
<211>  12
<212>  PRT
<213>  Artificial Sequence

<220>
<223>  Peptide

<400>  53

Trp Lys Asp Cys Glu Gly Ala Gly Lys Pro Leu Val
1               5                   10

<210>  54
<211>  13
<212>  PRT
<213>  Artificial Sequence

<220>
<223>  Peptide

<400>  54

Trp Lys Asp Glu Pro Ala Gly Lys Pro Leu Val Val Lys
1               5                   10


<210>  55
<211>  12
<212>  PRT
<213>  Artificial Sequence

<220>
<223>  Peptide

<400>  55

Trp Lys Asp Glu Ala Gly Pro Lys Pro Leu Val Lys
1               5                   10


<210>  56
<211>  14
<212>  PRT
<213>  Artificial Sequence

<220>
<223>  Peptide

<400>  56

Trp Lys Asp Glu Ala Gly Trp Ala Asp Lys Pro Leu Val Lys
1               5                   10


<210>  57
<211>  12
<212>  PRT
<213>  Artificial Sequence

<220>
<223>  Peptide

<400>  57

Trp Lys Asn Asp Glu Ala Gly Lys Pro Leu Val Lys
1               5                   10


<210>  58
<211>  9
<212>  PRT
<213>  Artificial Sequence

<220>

<223>  Peptide

<400>  58

Trp Lys Asp Ala Lys Pro Leu Val Lys
1               5

<210>  59
<211>  9
<212>  PRT
<213>  Artificial Sequence

<220>
<223>  Peptide

<400>  59

Trp Lys Glu Ala Gly Lys Pro Val Lys
1               5

<210>  60
<211>  10
<212>  PRT
<213>  Artificial Sequence

<220>
<223>  Peptide

<400>  60

Trp Lys Asp Glu Ala Lys Pro Leu Val Lys
1               5                   10

<210>  61
<211>  8
<212>  PRT
<213>  Artificial Sequence

<220>
<223>  Peptide

<400>  61

Trp Asp Glu Ala Gly Lys Pro Val
1               5

<210>  62
<211>  10
<212>  PRT
<213>  Artificial Sequence

<220>
<223>  Peptide

<400>  62

Trp Lys Asp Glu Ala Gly Lys Pro Val Lys
1               5                   10

<210>  63
<211>  9
<212>  PRT
<213>  Artificial Sequence

<220>
<223>  Peptide

<400>  63

Trp Asp Ala Gly Lys Pro Leu Val Lys
1               5


<210>  64
<211>  10
<212>  PRT
<213>  Artificial Sequence

<220>
<223>  Peptide

<400>  64

Trp Lys Asp Glu Ala Gly Lys Pro Leu Val
1               5                   10


<210>  65
<211>  8
<212>  PRT
<213>  Artificial Sequence

<220>
<223>  Peptide

<400>  65

Trp Glu Ala Gly Lys Pro Leu Val
1               5


<210>  66
<211>  6
<212>  PRT
<213>  Artificial Sequence

<220>
<223>  Peptide

<400>  66

Trp Lys Asp Glu Ala Gly
1               5


<210>  67
<211>  5
<212>  PRT
<213>  Artificial Sequence

<220>

<223>  Peptide

<400>  67

Trp Lys Asp Glu Ala
1               5


<210>  68
<211>  8
<212>  PRT
<213>  Artificial Sequence

<220>
<223>  Peptide

<400>  68

Lys Asp Glu Ala Gly Lys Pro Leu
1               5


<210>  69
<211>  5
<212>  PRT
<213>  Artificial Sequence

<220>
<223>  Peptide

<400>  69

Lys Asp Glu Ala Gly
1               5


<210>  70
<211>  7
<212>  PRT
<213>  Artificial Sequence

<220>
<223>  Peptide

<400>  70

Asp Glu Ala Gly Lys Pro Leu
1               5


<210>  71
<211>  5
<212>  PRT
<213>  Artificial Sequence

<220>
<223>  Peptide

<400>  71

Gly Lys Pro Leu Val
1               5

<210>  72
<211>  5
<212>  PRT
<213>  Artificial Sequence

<220>
<223>  Peptide

<400>  72

Asp Glu Ala Gly Lys
1               5


<210>  73
<211>  9
<212>  PRT
<213>  Artificial Sequence

<220>
<223>  Peptide

<400>  73

Trp Lys Asp Glu Ala Gly Lys Pro Leu
1               5


<210>  74

<400>  74
000

<210>  75

<400>  75
000

<210>  76
<211>  5
<212>  PRT
<213>  Artificial Sequence

<220>
<223>  Peptide

<400>  76

Lys Pro Leu Val Lys
1               5


<210>  77
<211>  4
<212>  PRT
<213>  Artificial Sequence

<220>
<223>  Peptide

<400>  77

Trp Lys Asp Glu
1

<210> 78
<211> 5
<212> PRT
<213> Artificial Sequence

<220>
<223> Peptide

<400> 78

Ala Gly Lys Pro Leu
1               5

<210> 79

<400> 79
000

<210> 80

<400> 80
000

<210> 81

<400> 81
000

<210> 82

<400> 82
000

<210> 83

<400> 83
000

<210> 84

<400> 84
000

<210> 85

<400> 85
000

<210> 86
<211> 11
<212> PRT
<213> Artificial Sequence

<220>
<221> source
<223> /note="Description of Artificial Sequence: Synthetic
      peptide"

<220>
<221> MOD_RES
<222> (1)..(1)
<223> D-Trp

<220>
<221> MOD_RES
<222> (2)..(2)
<223> D-Lys

<220>
<221> MOD_RES
<222> (5)..(5)
<223> D-Ala

<220>
<221> MOD_RES
<222> (10)..(10)
<223> D-Val

<220>
<221> MOD_RES
<222> (11)..(11)
<223> D-Lys

<400> 86
Xaa Xaa Asp Glu Xaa Gly Lys Pro Leu Xaa Xaa
1               5                   10

<210> 87
<211> 11
<212> PRT
<213> Artificial Sequence

<220>
<221> source
<223> /note="Description of Artificial Sequence: Synthetic
      peptide"

<220>
<221> SITE
<222> (1)..(5)
<223> /note="This sequence contains a thioether cyclization
      between the n-terminus and the Cys residue at position 5"

<220>
<221> MOD_RES
<222> (1)..(1)
<223> D-Trp

<220>
<221> MOD_RES
<222> (4)..(4)
<223> Glu(Me)

<220>
<221> MOD_RES
<222> (8)..(8)
<223> Pro(Me)

<220>

<221> MOD_RES
<222> (11)..(11)
<223> D-Lys

<220>
<221> source
<223> /note="C-term OH"

<400> 87
Xaa Lys Glu Xaa Cys Gly Lys Xaa Leu Val Xaa
1               5                   10


<210>  88
<211>  426
<212>  PRT
<213>  Homo sapiens

<400>  88

Met Ala Ile Ala Gln Leu Ala Thr Glu Tyr Val Phe Ser Asp Phe Leu
1               5                   10                  15

Leu Lys Glu Pro Thr Glu Pro Lys Phe Lys Gly Leu Arg Leu Glu Leu
            20                  25                  30

Ala Val Asp Lys Met Val Thr Cys Ile Ala Val Gly Leu Pro Leu Leu
        35                  40                  45

Leu Ile Ser Leu Ala Phe Ala Gln Glu Ile Ser Ile Gly Thr Gln Ile
    50                  55                  60

Ser Cys Phe Ser Pro Ser Ser Phe Ser Trp Arg Gln Ala Ala Phe Val
65                  70                  75                  80

Asp Ser Tyr Cys Trp Ala Ala Val Gln Gln Lys Asn Ser Leu Gln Ser
                85                  90                  95

Glu Ser Gly Asn Leu Pro Leu Trp Leu His Lys Phe Phe Pro Tyr Ile
            100                 105                 110

Leu Leu Leu Phe Ala Ile Leu Leu Tyr Leu Pro Pro Leu Phe Trp Arg
        115                 120                 125

Phe Ala Ala Ala Pro His Ile Cys Ser Asp Leu Lys Phe Ile Met Glu
    130                 135                 140

Glu Leu Asp Lys Val Tyr Asn Arg Ala Ile Lys Ala Ala Lys Ser Ala
145                 150                 155                 160

Arg Asp Leu Asp Met Arg Asp Gly Ala Cys Ser Val Pro Gly Val Thr
                165                 170                 175

Glu Asn Leu Gly Gln Ser Leu Trp Glu Val Ser Glu Ser His Phe Lys
            180                 185                 190

Tyr Pro Ile Val Glu Gln Tyr Leu Lys Thr Lys Lys Asn Ser Asn Asn
        195                 200                 205

Leu Ile Ile Lys Tyr Ile Ser Cys Arg Leu Leu Thr Leu Ile Ile Ile
    210                 215                 220

Leu Leu Ala Cys Ile Tyr Leu Gly Tyr Tyr Phe Ser Leu Ser Ser Leu
225                 230                 235                 240

Ser Asp Glu Phe Val Cys Ser Ile Lys Ser Gly Ile Leu Arg Asn Asp
                245                 250                 255

Ser Thr Val Pro Asp Gln Phe Gln Cys Lys Leu Ile Ala Val Gly Ile
            260                 265                 270

Phe Gln Leu Leu Ser Val Ile Asn Leu Val Val Tyr Val Leu Leu Ala
        275                 280                 285

Pro Val Val Val Tyr Thr Leu Phe Val Pro Phe Arg Gln Lys Thr Asp
    290                 295                 300

Val Leu Lys Val Tyr Glu Ile Leu Pro Thr Phe Asp Val Leu His Phe
305                 310                 315                 320

Lys Ser Glu Gly Tyr Asn Asp Leu Ser Leu Tyr Asn Leu Phe Leu Glu
                325                 330                 335

Glu Asn Ile Ser Glu Val Lys Ser Tyr Lys Cys Leu Lys Val Leu Glu
            340                 345                 350

Asn Ile Lys Ser Ser Gly Gln Gly Ile Asp Pro Met Leu Leu Leu Thr
        355                 360                 365

Asn Leu Gly Met Ile Lys Met Asp Val Val Asp Gly Lys Thr Pro Met
    370                 375                 380

Ser Ala Glu Met Arg Glu Glu Gln Gly Asn Gln Thr Ala Glu Leu Gln
385                 390                 395                 400

Gly Met Asn Ile Asp Ser Glu Thr Lys Ala Asn Asn Gly Glu Lys Asn
                405                 410                 415

Ala Arg Gln Arg Leu Leu Asp Ser Ser Cys
            420                 425

<210>  89
<211>  422
<212>  PRT
<213>  Homo sapiens

<400>  89

Met Ala Ile Ala Gln Leu Ala Thr Glu Tyr Val Phe Ser Asp Phe Leu
1               5                   10                  15

Leu Lys Glu Pro Thr Glu Pro Lys Phe Lys Gly Leu Arg Leu Glu Leu
            20                  25                  30

Ala Val Asp Lys Met Val Thr Cys Ile Ala Val Gly Leu Pro Leu Leu
        35                  40                  45

Leu Ile Ser Leu Ala Phe Ala Gln Glu Ile Ser Ile Gly Thr Gln Ile
    50                  55                  60

Ser Cys Phe Ser Pro Ser Ser Phe Ser Trp Arg Gln Ala Ala Phe Val
65                  70                  75                  80

Asp Ser Tyr Cys Trp Ala Ala Val Gln Gln Lys Asn Ser Leu Gln Ser
                85                  90                  95

Glu Ser Gly Asn Leu Pro Leu Trp Leu His Lys Phe Phe Pro Tyr Ile
            100                 105                 110

Leu Leu Leu Phe Ala Ile Leu Leu Tyr Leu Pro Pro Leu Phe Trp Arg
        115                 120                 125

Phe Ala Ala Ala Pro His Ile Cys Ser Asp Leu Lys Phe Ile Met Glu
    130                 135                 140

Glu Leu Asp Lys Val Tyr Asn Arg Ala Ile Lys Ala Ala Lys Ser Ala
145                 150                 155                 160

Arg Asp Leu Asp Met Arg Asp Gly Ala Cys Ser Val Pro Gly Val Thr
                165                 170                 175

Glu Asn Leu Gly Gln Ser Leu Trp Glu Val Ser Glu Ser His Phe Lys
            180                 185                 190

Tyr Pro Ile Val Glu Gln Tyr Leu Lys Thr Lys Lys Asn Ser Asn Asn
        195                 200                 205

Leu Ile Ile Lys Tyr Ile Ser Cys Arg Leu Leu Thr Leu Ile Ile Ile
    210                 215                 220

Leu Leu Ala Cys Ile Tyr Leu Gly Tyr Tyr Phe Ser Leu Ser Ser Leu
225                 230                 235                 240

Ser Asp Glu Phe Val Cys Ser Ile Lys Ser Gly Ile Leu Arg Asn Asp
                245                 250                 255

Ser Thr Val Pro Asp Gln Phe Gln Cys Lys Leu Ile Ala Val Gly Ile
            260                 265                 270

Phe Gln Leu Leu Ser Val Ile Asn Leu Val Val Tyr Val Leu Leu Ala
        275                 280                 285

Pro Val Val Val Tyr Thr Leu Phe Val Pro Phe Arg Gln Lys Thr Asp
    290                 295                 300

Val Leu Lys Val Tyr Glu Ile Leu Pro Thr Phe Asp Val Leu His Phe
305                 310                 315                 320

Lys Ser Glu Gly Tyr Asn Asp Leu Ser Leu Tyr Asn Leu Phe Leu Glu
                325                 330                 335

Glu Asn Ile Ser Glu Val Lys Ser Tyr Lys Cys Leu Lys Val Leu Glu
            340                 345                 350

Asn Ile Lys Ser Ser Gly Gln Gly Ile Asp Pro Met Leu Leu Leu Thr
        355                 360                 365

Asn Leu Gly Met Ile Lys Met Asp Val Val Asp Gly Lys Thr Pro Met
    370                 375                 380

Ser Ala Glu Met Arg Glu Glu Gln Gly Asn Gln Thr Ala Glu Leu Gln
385                 390                 395                 400

Asp Ser Glu Thr Lys Ala Asn Asn Gly Glu Lys Asn Ala Arg Gln Arg
                405                 410                 415

Leu Leu Asp Ser Ser Cys
            420